(19) Europäisches Patentamt
European Patent Office
Office européen des brevets

(11) **EP 3 906 323 B1**

(12) **EUROPEAN PATENT SPECIFICATION**

(45) Date of publication and mention
of the grant of the patent:
**31.12.2025 Bulletin 2026/01**

(21) Application number: **20700233.8**

(22) Date of filing: **02.01.2020**

(51) International Patent Classification (IPC):
*C12Q 1/6883* $^{(2018.01)}$      *G01N 33/50* $^{(2006.01)}$
*G01N 33/532* $^{(2006.01)}$

(52) Cooperative Patent Classification (CPC):
**C12Q 1/6883; G01N 33/5023; G01N 33/5047;**
**G01N 33/532;** C12Q 2600/142; C12Q 2600/158

(86) International application number:
**PCT/EP2020/050049**

(87) International publication number:
**WO 2020/141204 (09.07.2020 Gazette 2020/28)**

(54) **METHOD FOR IDENTIFYING AGENTS CAPABLE OF INDUCING RESPIRATORY SENSITIZATION AND ARRAY AND ANALYTICAL KITS FOR USE IN THE METHOD**

VERFAHREN ZUR IDENTIFIZIERUNG VON MITTELN ZUR INDUZIERUNG VON RESPIRATORISCHER SENSIBILISIERUNG SOWIE ANORDNUNG UND ANALYSEKITS ZUR VERWENDUNG BEI DEM VERFAHREN

PROCÉDÉ POUR IDENTIFIER DES AGENTS CAPABLES D'INDUIRE UNE SENSIBILISATION DES VOIES RESPIRATOIRES ET PUCE ET KITS ANALYTIQUES À UTILISER DANS CE PROCÉDÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.01.2019 GB 201900067**

(43) Date of publication of application:
**10.11.2021 Bulletin 2021/45**

(73) Proprietor: **SenzaGen AB**
**223 81 Lund (SE)**

(72) Inventors:
• **JOHANSSON, Sven Henrik**
**21158 Malmö (SE)**
• **GRADIN, Robin Mikael**
**87010 Älandsbro (SE)**

(74) Representative: **Potter Clarkson**
**Chapel Quarter**
**Mount Street**
**Nottingham NG1 6HQ (GB)**

(56) References cited:
**WO-A1-2016/083604      WO-A1-2018/122219**

• **CHARY A ET AL: "In vitro model for the prediction of respiratory sensitization", TOXICOLOGY LETTERS, ELSEVIER BIOMEDICAL PRESS, AMSTERDAM, NL, vol. 295, 12 September 2018 (2018-09-12), XP085476397, ISSN: 0378-4274, DOI: 10.1016/ J.TOXLET.2018.06.689**
• **ROBINSON MARK D ET AL: "A comparison of Affymetrix gene expression arrays", BMC BIOINFORMATICS, BIOMED CENTRAL, LONDON, GB, vol. 8, no. 1, 15 November 2007 (2007-11-15), pages 449, XP021031592, ISSN: 1471-2105**
• **CHARY ALINE ET AL: "Respiratory sensitization: toxicological point of view on the available assays", ARCHIVES OF TOXICOLOGY, SPRINGER, DE, vol. 92, no. 2, 16 October 2017 (2017-10-16), pages 803 - 822, XP036432293, ISSN: 0340-5761, [retrieved on 20171016], DOI: 10.1007/S00204-017-2088-5**

- MICHÈLE GOUTET ET AL: "Identification of contact and respiratory sensitizers according to IL-4 receptor expression and IL-2 production", TOXICOLOGY AND APPLIED PHARMACOLOGY, ACADEMIC PRESS, AMSTERDAM, NL, vol. 260, no. 2, 13 February 2012 (2012-02-13), pages 95 - 104, XP028478634, ISSN: 0041-008X, [retrieved on 20120221], DOI: 10.1016/J.TAAP.2012.02.009

- ALINE CHARY CHARY: "An in vitro coculture system for the detection of sensitization following aerosol exposure", ALTERNATIVES TO ANIMAL EXPERIMENTATION : ALTEX, 20 February 2019 (2019-02-20), DE, XP055681802, ISSN: 1868-596X, DOI: 10.14573/altex.1901241

**Description**

**Field of the invention**

The present invention relates to a method for identifying agents capable of inducing respiratory sensitization and arrays and analytical kits for use in such methods.

**Background**

Chemical sensitization, also referred to as chemical allergy, is a disease state induced by the human immune system in response to chemical sensitizers. This category of substances (often fragrances, cosmetics additives, dyes and metal ions) exercise their harmful effects by triggering a multitude of intricate cellular mechanisms, as they are often able to penetrate tissue. Sensitization occurs when T-cells learn to recognize a specific chemical sensitizer. Following subsequent exposure, T-cells react rapidly to induce a state of inflammation. This in turn leads to disease-associated symptoms, such as itching, blistering and tissue damage in case of skin contact, and coughing, wheezing and asthma-like symptoms in case of inhalation.

It is well recognized that the route of exposure may have an impact on the observed symptoms (Kimber et al., 2011). However, it is also becoming increasingly clear that chemical compounds may have intrinsic properties that preferentially lead to sensitization of the skin or the respiratory tract, also referred to as allergic contact dermatitis (ACD) and occupational asthma (OA), respectively (Dearman et al., 2011).

In both cases, safety assessments of chemicals have historically been carried out using animal experiments. While the current gold standard, the murine Local Lymph Node Assay (LLNA) (TG 429) tends to classify both kinds of chemical sensitizers as positive, it is inadequate in differentiating the two (Dearman et al., 2011). In addition, public opinion, concern for human environmental health and economic interests have led to legislations within the EU that prohibits the use of animal experiments to perform safety assessments on cosmetics and any ingredients thereof, a trend that is currently spreading both globally and across market and industry segments. Taken together, there is an urgent need to develop animal-free methods for assessment of chemical sensitizers.

To meet this demand, a lot of research during the last decade has focused on method development of so-called in vitro, in chemico and in silico assays, i.e. predictive methods that can classify tested chemicals as sensitizers or non-sensitizers without the use of animal experiments. While a number of assays for assessment of skin sensitizers have been proposed, some of which have undergone formal validation and are thus approved for industrial implementation (i.e. OECD TG 442C, 442D and 442E), the demand for an assay that accurately and specifically predicts and classifies chemical respiratory sensitizers remains unfulfilled.

A contributing factor to the absence of predictive methods for chemical respiratory sensitizers is the large knowledge gap that currently prohibits a detailed understanding of the immunobiological mechanisms involved in respiratory sensitization. Compared to the case of skin sensitization, an adverse outcome pathway (AOP) is not readily available. However, work to create such an AOP is progressing and many fundamental steps of the mechanistic pathway are largely agreed upon (e.g. Kimber et al., 2014, Sullivan et al., 2017).

Briefly, the initiating molecular event and subsequent key events are largely analogous to the AOP of skin sensitization, with a few key areas of uncertainties, as well as the obvious discrepancy relating to organ-specific reallocation of cellular events to the periphery of the respiratory tract. However, while elicitation typically will require respiratory exposure, it should be noted that respiratory sensitization can also occur through skin exposure (Kimber et al., 2002), further substantiating the notion that skin and respiratory sensitizers are intrinsically different, preferentially leading to one adverse outcome or the other, and very rarely both.

Similar to the case of skin sensitization, the proposed AOP is suggested to start with a covalent protein binding, likely to lysine nucleophiles in the lung or skin after respiratory or dermal exposure to a low molecular weight organic chemical. This protein binding causes the activation of stress response pathways and cellular danger signals including oxidative stress, cytokines and chemokines released by epithelial cells, leading to dendritic cell (DC) maturation and migration to the draining lymph nodes. Haptens can also contribute to DC activation directly. Antigen-presenting DCs in the draining lymph nodes signal activation and maturation of T cells which characterize the sensitization phase, resulting in chemical respiratory allergy.

Thus, the AOP for chemical respiratory sensitization includes a molecular initiation event (key event (KE) 1), cellular inflammatory responses in lung epithelial (KE 2) and DCs (KE 3), and organ responses (e.g. T cell responses (KE 4)). While it is believed that respiratory sensitizers preferentially induce a Th2-type immune response, as opposed to the Th1 and cytotoxic T-cells primarily induced by skin sensitizers, a key area of uncertainty involves the exact location, involved cellular subsets and molecular mechanism by which this Th2-skewing occur (Paul & Zhu, 2010). Furthermore, whether IgE-antibodies are required for elicitation of adverse effects is not fully understood (Isola et al., 2008). However, it is hypothesized that DCs are involved and that Th2-skewing occurs in association with antigen-presentation, through the co-stimulatory profile exhibited by DCs at the immunological synapse. For these reasons, an in vitro cell system of DCs are candidate targets for assay development.

The Genomic Allergen Rapid Detection (GARD™) platform has previously demonstrated the capacity to classify respiratory sensitizers using different gene signatures each based on more than 300 biomarkers (Forreryd et al., 2015, WO 2013/160882; WO 2016/083604). However, there is a continuing and urgent need to establish accurate and reliable animal-free in vitro assays for specifically identifying respiratory sensitizers.

Chary A, et al Toxicology Letters 195 (2018) discloses an in vitro model for the prediction of respiratory sensitization. WO 2018/122219 discloses a three-dimensional *in vitro* alveolar lung model, process for preparing said model, and its use for determining and/or predicting the sensitizing effects of inhalable products. Robinson et al BMC Bioinformatics 8,1; 449 (2007) discloses a comparison of Affymetrix gene expression arrays.

## Disclosure of the invention

The inventors have now produced a novel cell-based testing strategy for assessment of respiratory sensitizers based on a new genomic biomarker signature surprisingly comprising a new small set of genes which can be used in combination as an alternative to animal testing. The inventors demonstrate the functionality of the assay, henceforth referred to as "GARDair", by providing classification data generated from classifications of samples in an external test data set.

Accordingly, a first aspect of the present invention provides an *in vitro* method for identifying agents capable of inducing respiratory sensitization in a mammal comprising or consisting of the steps of:

(a) providing a population of dendritic cells or a population of dendritic-like cells;

(b) exposing the cells provided in step (a) to a test agent; and

(c) measuring in the cells of step (b) the expression of all of the biomarkers listed in Table A;

wherein the expression of the biomarkers measured in step (c) is indicative of the respiratory sensitizing effect of the test agent of step (b).

Also disclosed herein, one or more of the biomarkers for which the expression is measured in step (c) is selected from the group defined in Table A(i).

Also disclosed herein step (c) comprises or consists of measuring the expression of one or more biomarkers selected from the group defined in Table A(i), for example, 2, 3, 4, 5, 6, 7, 8, 9, 10, 11, 12, 13, 14, 15, 16, 17, 18, 19, 20, 21, 22, 23, 24, or 25 of the biomarkers listed in Table A(i). For example, step (c) may comprise or consist of measuring the expression of all of the biomarkers listed in Table A(i).

Also disclosed herein the method may include or exclude measuring the expression of CRLF2. The method may include or exclude measuring the expression of FSCN1. The method may include or exclude measuring the expression of AES. The method may include or exclude measuring the expression of ALOX5AP. The method may include or exclude measuring the expression of RAB27B. The method may include or exclude measuring the expression of ZFP36L1. The method may include or exclude measuring the expression of SLC44A2. The method may include or exclude measuring the expression of ATL1. The method may include or exclude measuring the expression of FAM30A. The method may include or exclude measuring the expression of CTSH. The method may include or exclude measuring the expression of NINJ1. The method may include or exclude measuring the expression of RALGAPA2. The method may include or exclude measuring the expression of RNF220. The method may include or exclude measuring the expression of OSBPL3. The method may include or exclude measuring the expression of CACNA2D2. The method may include or exclude measuring the expression of HNRNPC. The method may include or exclude measuring the expression of PIK3C3. The method may include or exclude measuring the expression of HOPX. The method may include or exclude measuring the expression of VCAN. The method may include or exclude measuring the expression of RUFY1. The method may include or exclude measuring the expression of GNA15. The method may include or exclude measuring the expression of ADAM8. The method may include or exclude measuring the expression of NRIP1. The method may include or exclude measuring the expression of CTCF. The method may include or exclude measuring the expression of PLCXD1.

The method may include or exclude measuring the expression of MYCN. The method may include or exclude measuring the expression of IL7R. The method may include or exclude measuring the expression of RALA.

Also disclosed herein step (c) comprises or consists of measuring the expression of one or more biomarkers selected from the group defined in in Table A(ii), for example, 2, or 3 of the biomarkers listed in Table A(ii). For example, step (c) may comprise or consist of measuring the expression of all of the biomarkers listed in Table A(ii).

Also disclosed herein CRLF2 is included in Table A(ii) and not in Table A(i).

Also disclosed herein step (c) comprises or consists of measuring the expression of three or more of the biomarkers selected from the group defined in in Table A, for example, 3, 4, 5, 6, 7, 8, 9, 10, 11, 12, 13, 14, 15, 16, 17, 18, 19, 20, 21, 22, 23, 24, 25, 26, 27, or 28 of the biomarkers listed in Table A. For example, step (c) may comprise or consist of measuring the expression of all of the biomarkers listed in Table A.

Thus, also disclosed herein the expression of all of the biomarkers defined in Table A(i) and/or all of the biomarkers defined in Table A(ii) may be measured in step (c). Hence, the method may comprise or consist of measuring in step (c) all of the biomarkers defined in Table A.

Also disclosed herein step (c) comprises or consists of measuring the expression of each of the following biomarkers: CRLF2, FSCN1.

Also disclosed herein step (c) comprises or consists of measuring the expression of each of the following biomarkers: CRLF2, FSCN1, AES.

Also disclosed herein step (c) comprises or consists of measuring the expression of each of the following biomarkers: CRLF2, FSCN1, AES, ALOX5AP.

Also disclosed herein step (c) comprises or consists of measuring the expression of each of the following biomarkers: CRLF2, FSCN1, AES, ALOX5AP, RAB27B.

Also disclosed herein step (c) comprises or consists of measuring the expression of each of the following biomarkers: CRLF2, IL7R.

Also disclosed herein step (c) comprises or consists of measuring the expression of each of the following biomarkers: CRLF2, FSCN1, AES, ALOX5AP, RAB27B, MYCN, ZFP36L1, SLC44A2, ATL1, FAM30A.

Also disclosed herein step (c) comprises or consists of measuring the expression of each of the following biomarkers: CRLF2, FSCN1, AES, ALOX5AP, RAB27B, MYCN, ZFP36L1, SLC44A2, ATL1, FAM30A, CTSH, NINJ1, RALGAPA2, RNF220, OSBPL3,

Also disclosed herein step (c) comprises or consists of measuring the expression of each of the following biomarkers: CRLF2, FSCN1, AES, ALOX5AP, RAB27B, MYCN, ZFP36L1, SLC44A2, ATL1, FAM30A, CTSH, NINJ1, RALGAPA2, RNF220, OSBPL3, CACNA2D2, HNRNPC, PIK3C3, IL7R.

Also disclosed herein step (c) comprises or consists of measuring the expression of each of the following biomarkers: CRLF2, FSCN1, AES, ALOX5AP, RAB27B, MYCN, ZFP36L1, SLC44A2, ATL1, FAM30A, CTSH, NINJ1, RALGAPA2, RNF220, OSBPL3, CACNA2D2, HNRNPC, PIK3C3, IL7R, HOPX.

Also disclosed herein step (c) comprises or consists of measuring the expression of each of the following biomarkers: CRLF2, FSCN1, AES, ALOX5AP, RAB27B, MYCN, ZFP36L1, SLC44A2, ATL1, FAM30A, CTSH, NINJ1, RALGAPA2, RNF220, OSBPL3, CACNA2D2, HNRNPC, PIK3C3, IL7R, HOPX, VCAN, RALA, RUFY1, GNA15, ADAM8, NRIP1, CTCF, PLCXD1.

By "expression" we mean the presence, level, and/or amount of the biomarker.

By "biomarker" we include any biological molecule, or component or fragment thereof, the measurement of which can provide information useful in determining the if a test agent is a respiratory sensitizer. Thus, in the context of Table A, the biomarker may be a nucleic acid molecule, such as a mRNA or cDNA. Alternatively, the biomarker may be a protein encoded by the nucleic acid molecule, or carbohydrate moiety, antigenic component or fragment thereof.

In an additional or alternative embodiment the method comprises the further steps of:

d) exposing a separate population of the dendritic cells or dendritic-like cells to one or more negative control agent that is not a respiratory sensitizer in a mammal; and
e) measuring in the cells of step (d) the expression of the biomarkers measured in step (c)

wherein the test agent is identified as a respiratory sensitizer in the event that the expression of the biomarkers measured in step (e) differs from the expression of the biomarkers measured in step (c).

In an additional or alternative embodiment DMSO may be used as the negative control. A vehicle control may be used as the negative control agent. The vehicle control may comprise DMSO.

In an additional or alternative embodiment unstimulated cells may be used as the negative control. By "unstimulated cells" we include or mean cells which have not been exposed to any test agent. In other words, the separate population of cells in step (d) is not exposed to a test agent. In an additional or alternative embodiment unstimulated cells may be used as a reference sample for alignment of data sets for normalization purposes.

In an additional or alternative embodiment the expression of the biomarkers measured in step (c) is measured in the cells provided in step (a) prior to and following exposure to the test agent, and wherein the difference in expression between the biomarkers prior to and following exposure to the test agent is indicative of the sensitizing effect of the test agent of step (b). Hence, the cells provided in step (a) may provide both the negative control and the test result.

By "differs from the expression of the biomarkers measured in step (c)" and "difference in expression" we include that the presence and or amount in a first sample (e.g., a test agent sample) differs from that of a second sample (e.g., a control agent sample).

For example, the presence and/or amount in the test sample may differ from that of the one or more negative control sample in a statistically significant manner. Preferably the expression of the biomarkers in the cell population exposed to the test agent is:

less than or equal to 80% of that of the cell population exposed to the negative control agent, for example, no more than 79%, 78%, 77%, 76%, 75%, 74%, 73%, 72%, 71%, 70%, 69%, 68%, 67%, 66%, 65%, 64%, 63%, 62%, 61%, 60%, 59%, 58%, 57%, 56%, 55%, 54%, 53%, 52%, 51%, 50%, 49%, 48%, 47%, 46%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2%, 1% or 0% of that of the cell population exposed to the negative control or negative control agent; or

at least 120% of that of the cell population exposed to the negative control agent, for example, at least 121%, 122%, 123%, 124%, 125%, 126%, 127%, 128%, 129%, 130%, 131%, 132%, 133%, 134%, 135%, 136%, 137%, 138%, 139%, 140%, 141%, 142%, 143%, 144%, 145%, 146%, 147%, 148%, 149%, 150%, 151%, 152%, 153%, 154%, 155%, 156%, 157%, 158%, 159%, 160%, 161%, 162%, 163%, 164%, 165%, 166%, 167%, 168%, 169%, 170%, 171%, 172%, 173%, 174%, 175%, 176%, 177%, 178%, 179%, 180%, 181%, 182%, 183%, 184%, 185%, 186%, 187%, 188%, 189%, 190%, 191%, 192%, 193%, 194%, 195%, 196%, 197%, 198%, 199%, 200%, 225%, 250%, 275%, 300%, 325%, 350%, 375%, 400%, 425%, 450%, 475% or at least 500% of that of the cell population exposed to the negative control or negative control agent

By "differs from the expression of the biomarkers measured in step (c)" we alternatively or additionally include that the test sample is classified as belonging to a different group as the one or more negative control sample. For example, where an SVM is used, the test sample is on the other side of the decision value threshold as the one or more negative control sample (e.g., if the test agent is classified as a respiratory sensitizer if one or more test (or replicate thereof) has an SVM decision value of ≤0, then the one or more positive control samples (or the majority thereof) should also have an SVM decision value of ≤0).

In an additional or alternative embodiment, the one or more negative control agent provided in step (d) is/are selected from the group consisting of: DMSO; unstimulated cells; cell media; vehicle control; distilled water.

In an additional or alternative embodiment, the one or more negative control agent may comprise or consist of one or more agent selected from the group consisting of DMSO; 1-Butanol; 2-Aminophenol; 2-Hydroxyethyl acrylate; 2-nitro-1,4-Phenylenediamine; 4-Aminobenzoic acid; Chlorobenzene; Dimethyl formamide; Ethyl vanillin; Formaldehyde; Geraniol; Hexylcinnamic aldehyde; Isopropanol; Kathon CG*; Methyl salicylate; Penicillin G; Propylene glycol; Potassium Dichromate; Potassium permanganate; Tween 80; Zinc sulphate; 2-Mercaptobenzothiazole; 4-Hydroxybenzoic acid; Benzaldehyde; Octanoic acid; Cinnamyl alcohol; Diethyl phthalate; DNCB; Eugenol; Glycerol; Glyoxal; Isoeugenol; Phenol; PPD; Resorcinol; Salicylic acid; SDS; and Chlorobenzene.

In an additional or alternative particular embodiment the one or more negative control agent may comprise or consist of DMSO and/or Chlorobenzene.

In an additional or alternative embodiment, the one or more negative control agent may comprise or consist of one or more agent selected from the group consisting of those non-sensitizers and/or non-respiratory sensitizers listed in Table 1 and/or Table 3.

The negative control agent may be a solvent for use with the test or control agents in the method of the invention.

The method may comprise or consist of the use of at least 2 negative control agents (i.e. non-sensitizing agents), for example, at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or at least 100 negative control agents.

Alternatively or additionally, the expression of the biomarkers measured in step (b) of the dendritic cells or dendritic-like cells prior to test agent exposure is used as a negative control.

In an additional or alternative embodiment the method comprises the further steps of:

f) exposing a separate population of the dendritic cells or dendritic-like cells to one or more positive control agent that is a respiratory sensitizer in a mammal; and
g) measuring in the cells of step (f) the expression of the biomarkers measured in step (c)

wherein the test agent is identified as a respiratory sensitizer in the event that the expression of the biomarkers measured in step (f) corresponds to the expression of the biomarkers measured in step (c).

By "corresponds to the expression of the biomarkers measured in step (c)" we mean the expression of the biomarkers in the cell population exposed to the test agent is identical to, or does not differ significantly from, that of the cell population exposed to the one more positive control agent. Preferably the expression of the biomarkers in the cell population exposed to the test agent is between 81% and 119% of that of the cell population exposed to the one more positive control agent, for example, greater than or equal to 82%, 83%, 84%, 85%, 86%, 87%, 88%, 89%, 90%, 91%, 92%, 93%, 94%, 95%, 96%, 97%, 98% or 99% of that of the cell population exposed to the one more positive control agent, and less than or equal to 101%, 102%, 103%, 104%, 105%, 106%, 107%, 108%, 109%, 110%, 111%, 112%, 113%, 114%, 115%, 116%, 117%, 118% or 119% of that of the cell population exposed to the one more positive control agent.

By "corresponds to the expression of the biomarkers measured in step (c)" we alternatively or additionally include that the test sample is classified as belonging to the same group as the one or more positive control sample. For example, where an SVM is used, the test sample is on the same side of the decision value threshold as the one or more positive control sample (e.g., if the test agent is classified as a respiratory sensitizer if one or more test (or replicate thereof) has an SVM decision value of >0, then the one or more positive control samples (or the majority thereof) should also have an SVM decision value of >0).

In an additional or alternative embodiment, the one or more positive control agent provided in step (f) comprises or consists of one or more agent selected from the group consisting of: Ammonium hexachloroplatinate; Ammonium persulfate; Ethylenediamine; Glutaraldehyde; Hexamethylen diisocyanate; Maleic Anhydride; Methylene diphenol diisocyanate; Phtalic Anhydride; Toluendiisocyanate; Trimellitic anhydride; Chloramine-T hydrate; Isophorone diisocyanate; Piperazine; Reactive orange 16; Maleic anhydride; Phenyl isocyanate (MDI); Phthalic anhydride; Toluene diisocyanate; and Trimelitic anhydride..

In an additional or alternative embodiment, the one or more positive control agent provided in step (f) comprises or consists of one or more agent selected from the group consisting of: Reactive Orange 16; Piperazine; Chloramine T; and Trimellitic Anhydride.

In an additional or alternative embodiment, the one or more positive control agent provided in step (f) comprises or consists of one or more agent selected from the group consisting of: Reactive Orange 16; and Piperazine.

In an additional or alternative embodiment, the one or more positive control agent may comprise or consist of one or more agent selected from the group consisting of those respiratory sensitizers listed in Table 1 and/or Table 3.

In an additional or alternative embodiment, the one or more positive control agent may comprise or consist of Methylene diphenol diisocyanate.

The method may comprise or consist of the use of at least 2 positive control (i.e. sensitizing agents), for example, at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or at least 100 positive control agents.

In an additional or alternative embodiment, the method is indicative of the sensitization potency of the agent to be tested. For example, the method may be used to predict the relative sensitization potency of a test agent compared to a positive control and/or compared to one or more additional test agents.

In an additional or alternative embodiment the method comprises the further step of:

(h) identifying if the test agent is a respiratory sensitizer.

Hence, in one embodiment, the method is indicative of whether the test agent is or is not a respiratory sensitizing agent. In an alternative or additional embodiment, the method is indicative of the relative respiratory sensitizing potency of the test agent.

Thus, in one embodiment, the method is indicative of the sensitizer potency of the test agent (i.e., that the test agent is either, a non-sensitizer, a weak sensitizer, a moderate sensitizer, a strong sensitizer or an extreme sensitizer). Preferably, the decision value and distance in PCA correlates with sensitizer potency.

Alternatively or additionally, test agent potency may be determined by, in step (d), providing:

(i) one or more extreme respiratory sensitizer positive control agent;
(ii) one or more strong respiratory sensitizer positive control agent;
(iii) one or more moderate respiratory sensitizer positive control agent; and/or
(iv) one or more weak respiratory sensitizer positive control agent,

wherein the test agent is identified as an extreme respiratory sensitizer in the event that the presence and/or amount in the test sample of the biomarker measured in step (c) corresponds to the presence and/or amount in the extreme positive control sample (where present) of the biomarker measured in step (e); and/or is different from the presence and/or amount in the strong, moderate, weak and/or negative control sample (where present) of the biomarkers measured in step (e) and/or (g),

wherein the test agent is identified as a strong respiratory sensitizer in the event that the presence and/or amount in the test sample of the biomarker measured in step (c) corresponds to the presence and/or amount in the strong positive control sample (where present) of the biomarker measured in step (e); and/or is different from the presence and/or amount in the extreme, moderate, weak and/or negative control sample (where present) of the biomarkers measured in step (e) and/or (g),

wherein the test agent is identified as a moderate respiratory sensitizer in the event that the presence and/or amount in the test sample of the biomarker measured in step (c) corresponds to the presence and/or amount in the moderate positive control sample (where present) of the biomarker measured in step (e); and/or is different from the presence and/or amount in the extreme, strong, weak and/or negative control sample (where present) of the biomarkers measured in step (e) and/or (g), and

wherein the test agent is identified as a weak respiratory sensitizer in the event that the presence and/or amount in the test sample of the biomarker measured in step (c) corresponds to the presence and/or amount in the weak positive control sample (where present) of the biomarker measured in step (e); and/or is different from the presence and/or amount in the extreme, strong, moderate and/or negative control sample (where present) of the biomarkers measured in step (e) and/or (g).

Hence, step (d) may comprise or consist of providing the following categories of respiratory sensitizer positive control:

(a) extreme, strong, moderate and weak;
(b) strong, moderate and weak;
(c) extreme, moderate and weak;
(d) extreme, strong and moderate;
(e) extreme and strong;
(f) strong and moderate;
(g) moderate and weak;
(h) strong and weak;
(i) extreme and moderate;
(j) extreme and weak;
(k) extreme;
(l) strong;
(m) moderate;
(n) weak.

Negative and positive controls may be classified as respiratory non-sensitizers or respiratory sensitizers, respectively, based on clinical observations in humans.

Alternatively or additionally the method may comprise comparing the expression of the biomaker measured in step (c) with one or more predetermined reference value representing the expression of the biomarker measured in step (e) and/or step (g).

By appropriate selection of all of the biomarkers in Table A, optionally in conjunction with one or more further biomarkers, the methods of the invention exhibit high predictive accuracy for the identification of respiratory sensitizers.

Generally, respiratory sensitizing agents are determined with an ROC AUC of at least 0.55, for example with an ROC AUC of at least, 0.60, 0.65, 0.70, 0.75, 0.80, 0.85, 0.90, 0.95, 0.96, 0.97, 0.98, 0.99 or with an ROC AUC of 1.00. Preferably, respiratory sensitizing agents are determined with an ROC AUC of at least 0.85, and most preferably with an ROC AUC of 1.

The identification may be performed using any suitable statistical method or machine learning algorithm known in the art, such as Random Forest (RF), Support Vector Machine (SVM), Principal Component Analysis (PCA), ordinary least squares (OLS), partial least squares regression (PLS), orthogonal partial least squares regression (O-PLS) and other multivariate statistical analyses (e.g., backward stepwise logistic regression model). For a review of multivariate statistical analysis see, for example, Schervish, Mark J. (November 1987). "A Review of Multivariate Analysis". Statistical Science 2 (4): 396-413.

Preferably, Support Vector Machine (SVM) is used.

Typically, respiratory sensitizers are identified using a support vector machine (SVM), such as those available from http://cran.r-project.org/web/packages/e1071/index.html (e.g. e1071 1.5-24). However, any other suitable means may also be used. SVMs may also be used to determine the ROC AUCs of biomarker signatures comprising or consisting of one or more Table A biomarkers as defined herein.

Support vector machines (SVMs) are a set of related supervised learning methods used for classification and regression. Given a set of training examples, each marked as belonging to one of two categories, an SVM training algorithm builds a model that predicts whether a new example falls into one category or the other. Intuitively, an SVM model is a representation of the examples as points in space, mapped so that the examples of the separate categories are divided by a clear gap that is as wide as possible. New examples are then mapped into that same space and predicted to belong to a category based on which side of the gap they fall on.

More formally, a support vector machine constructs a hyperplane or set of hyperplanes in a high or infinite dimensional space, which can be used for classification, regression or other tasks. Intuitively, a good separation is achieved by the hyperplane that has the largest distance to the nearest training datapoints of any class (so-called functional margin), since in general the larger the margin the lower the generalization error of the classifier. For more information on SVMs, see for example, Burges, 1998, Data Mining and Knowledge Discovery, 2:121-167.

In one embodiment of the invention, the SVM is 'trained' prior to performing the methods of the invention using biomarker profiles of known agents (namely, known respiratory sensitizers or non-sensitizers). By running such training samples, the SVM is able to learn what biomarker profiles are associated with agents capable of inducing respiratory sensitization. Once the training process is complete, the SVM is then able to predict whether or not the biomarker sample tested is from a respiratory sensitizer or non-sensitizer.

Decision values for individual SVMs can be determined by the skilled person on a case-by-case basis. In one embodiment, the test agent is classified as a respiratory sensitizer if one or more test (or replicate thereof) have an SVM decision value of >0. In one embodiment, the test agent is classified as a respiratory non-sensitizer if one or more test (or replicate thereof) have an SVM decision value of ≤0. This allows test agents to be classified as a respiratory sensitizer or non-sensitizer.

However, this training procedure can be by-passed by pre-programming the SVM with the necessary training parameters. For example, respiratory sensitizers can be identified according to the known SVM parameters using the SVM algorithm described in the Example, based on the measurement of two or more of the biomarkers listed in Table A.

It will be appreciated by skilled persons that suitable SVM parameters can be determined for any combination of the biomarkers listed Table A by training an SVM machine with the appropriate selection of data (i.e. biomarker measurements from cells exposed to known respiratory sensitizers and/or non-sensitizers). Alternatively, the Table A biomarkers may be used to identify respiratory sensitizers according to any other suitable statistical method known in the art.

Alternatively, the Table A data may be used to identify agents capable of inducing respiratory sensitization according to any other suitable statistical method known in the art (e.g., ANOVA, ANCOVA, MANOVA, MANCOVA, Multivariate regression analysis, Principal components analysis (PCA), Factor analysis, Canonical correlation analysis, Canonical correlation analysis, Redundancy analysis Correspondence analysis (CA; reciprocal averaging), Multidimensional scaling, Discriminant analysis, Linear discriminant analysis (LDA), Clustering systems, Recursive partitioning and Artificial neural networks).

Preferably, the methods of the invention have an accuracy of at least 60%, for example, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% accuracy. In a preferred embodiment, the methods of the invention have an accuracy of at least 89%.

Preferably, the methods of the invention have a sensitivity of at least 60%, for example, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% sensitivity. In a preferred embodiment, the methods of the invention have a sensitivity of at least 89%.

Preferably, the methods of the invention have a specificity of at least 60%, for example, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% specificity. In a preferred embodiment, the methods of the invention have a specificity of 89%.

By "accuracy" we mean the proportion of correct outcomes of a method, by "sensitivity" we mean the proportion of all positive agents that are correctly classified as positives, and by "specificity" we mean the proportion of all negative agents that are correctly classified as negatives.

In a preferred embodiment, step (c) comprises or consists of measuring the expression of a nucleic acid molecule of one or more of the biomarkers. The nucleic acid molecule may be a DNA molecule or a cDNA molecule or an mRNA molecule. Preferably, the nucleic acid molecule is an mRNA molecule. However, the nucleic acid molecule may be a cDNA molecule.

In one embodiment the measurement of the expression of one or more of the biomarkers in step (c) is performed using a method selected from the group consisting of Southern hybridisation, Northern hybridisation, polymerase chain reaction (PCR), reverse transcriptase PCR (RT-PCR), quantitative real-time PCR (qRT-PCR), nanoarray, microarray, macroarray, autoradiography and *in situ* hybridisation. Preferably, the expression of one or more biomarker(s) is measured using a DNA microarray.

In an additional or alternative embodiment the one or more biomarkers measured in step (c) is measured using an array (e.g., a DNA array). In an additional or alternative embodiment the one or more biomarkers measured in step (c) is measured using a whole genome array (e.g., the Affymetrix Human Gene 1.0 ST array or Affymetrix Human Gene 2.0 ST array). In an alternative or additional embodiment, the Nanostring nCounter® system is used (e.g., custom Nanostring nCounter® code sets based on selection from a whole genome array (e.g., Affymetrix Human Gene 1.0 ST array or Affymetrix Human Gene 2.0 ST array). Such systems can be used according to the manufacturer's instructions, using recommended kits and reagents. In an additional or alternative embodiment the code set contains probes for one or more of the 28 genes defined in Table A.

The method may comprise measuring the expression of one or more biomarkers in step (c) using one or more binding moieties, each capable of binding selectively to a nucleic acid molecule encoding one of the biomarkers identified in Table A. Preferably, the method comprises measuring the expression of two or more biomarkers in step (c) using two or more binding moieties, each capable of binding selectively to a nucleic acid molecule encoding one of the biomarkers identified in Table A. For example, the expression of any particular combination of biomarkers described above may be measured using an equivalent combination of binding moieties capable of binding selectively to each of those biomarkers.

In one embodiment the one or more binding moieties each comprise or consist of a nucleic acid molecule. In a further embodiment the one or more binding moieties each comprise or consist of DNA, RNA, PNA, LNA, GNA, TNA or PMO. Preferably, the one or more binding moieties each comprise or consist of DNA. In one embodiment, the one or more binding moieties are 5 to 100 nucleotides in length. However, in an alternative embodiment, they are 15 to 35 nucleotides in length.

The one or more binding moieties may comprise or consist of one or more probe from the Human Gene 1.0 ST Array (Affymetrix, Santa Clara, CA, USA). Probe identification numbers are provided in Table A herein.

Suitable binding agents (also referred to as binding molecules or binding moieties) may be selected or screened from a library based on their ability to bind a given nucleic acid, protein or amino acid motif, as discussed below.

In a preferred embodiment, the binding moiety comprises a detectable moiety.

By a "detectable moiety" we include a moiety which permits its presence and/or relative amount and/or location (for example, the location on an array) to be determined, either directly or indirectly.

Suitable detectable moieties are well known in the art.

For example, the detectable moiety may be a fluorescent and/or luminescent and/or chemiluminescent moiety which, when exposed to specific conditions, may be detected. Such a fluorescent moiety may need to be exposed to radiation (*i.e.* light) at a specific wavelength and intensity to cause excitation of the fluorescent moiety, thereby enabling it to emit detectable fluorescence at a specific wavelength that may be detected.

Alternatively, the detectable moiety may be an enzyme which is capable of converting a (preferably undetectable) substrate into a detectable product that can be visualised and/or detected. Examples of suitable enzymes are discussed in more detail below in relation to, for example, ELISA assays.

The detectable moiety may be a radioactive moiety and comprise or consists of a radioactive atom. The radioactive atom may be selected from the group consisting of technetium-99m, iodine-123, iodine-125, iodine-131, indium-111, fluorine-19, carbon-13, nitrogen-15, oxygen-17, phosphorus-32, sulphur-35, deuterium, tritium, rhenium-186, rhenium-188 and yttrium-90.

Hence, the detectable moiety may be selected from the group consisting of: a fluorescent moiety; a luminescent moiety; a chemiluminescent moiety; a radioactive moiety (for example, a radioactive atom); or an enzymatic moiety.

Clearly, the agent to be detected (such as, for example, the one or more biomarkers in the test sample and/or control sample described herein and/or an antibody molecule for use in detecting a selected protein) must have sufficient of the appropriate atomic isotopes in order for the detectable moiety to be readily detectable.

In an alternative preferred embodiment, the detectable moiety of the binding moiety is a fluorescent moiety.

The radio- or other labels may be incorporated into the biomarkers present in the samples of the methods of the invention and/or the binding moieties used in the method of the invention in known ways. For example, if the binding agent is a polypeptide it may be biosynthesised or may be synthesised by chemical amino acid synthesis using suitable amino acid precursors involving, for example, fluorine-19 in place of hydrogen. Labels such as $^{99m}$Tc, $^{123}$I, [186]Rh, [188]Rh and $^{111}$In can, for example, be attached *via* cysteine residues in the binding moiety. Yttrium-90 can be attached via a lysine residue. The IODOGEN method (Fraker et al (1978) Biochem. Biophys. Res. Comm. 80, 49-57) can be used to incorporate [123]I. Reference ("Monoclonal Antibodies in Immunoscintigraphy", J-F Chatal, CRC Press, 1989) describes other methods in detail. Methods for conjugating other detectable moieties (such as enzymatic, fluorescent, luminescent, chemiluminescent or radioactive moieties) to proteins are well known in the art.

It will be appreciated by persons skilled in the art that biomarkers in the sample(s) to be tested may be labelled with a moiety which indirectly assists with determining the presence, amount and/or location of said proteins. Thus, the moiety may constitute one component of a multicomponent detectable moiety. For example, the biomarkers in the sample(s) to be tested may be labelled with biotin, which allows their subsequent detection using streptavidin fused or otherwise joined to a detectable label.

The method provided in the first aspect of the present invention may comprise or consist of, in step (c), determining the expression of the protein of one or more biomarker defined in Table A. The method may comprise measuring the expression of one or more biomarkers in step (c) using one or more binding moieties each capable of binding selectively to one of the biomarkers identified in Table A. The one or more binding moieties may comprise or consist of an antibody or an antigen-binding fragment thereof such as a monoclonal antibody or fragment thereof.

The term "antibody" includes any synthetic antibodies, recombinant antibodies or antibody hybrids, such as but not limited to, a single-chain antibody molecule produced by phage-display of immunoglobulin light and/or heavy chain variable and/or constant regions, or other immunointeractive molecules capable of binding to an antigen in an immunoassay format that is known to those skilled in the art.

We also include the use of antibody-like binding agents, such as affibodies and aptamers.

A general review of the techniques involved in the synthesis of antibody fragments which retain their specific binding sites is to be found in Winter & Milstein (1991) Nature 349, 293-299.

Additionally, or alternatively, one or more of the first binding molecules may be an aptamer (see Collett et al., 2005, Methods 37:4-15).

Molecular libraries such as antibody libraries (Clackson et al, 1991, Nature 352, 624-628; Marks et al, 1991, J Mol Biol 222(3): 581-97), peptide libraries (Smith, 1985, Science 228(4705): 1315-7), expressed cDNA libraries (Santi et al (2000) J Mol Biol 296(2): 497-508), libraries on other scaffolds than the antibody framework such as affibodies (Gunneriusson et al, 1999, Appl Environ Microbiol 65(9): 4134-40) or libraries based on aptamers (Kenan et al, 1999, Methods Mol Biol 118, 217-31) may be used as a source from which binding molecules that are specific for a given motif are selected for use in the methods of the invention.

The molecular libraries may be expressed *in vivo* in prokaryotic cells (Clackson *et al,* 1991, op. *cit.;* Marks *et al,* 1991, *op. cit.*) or eukaryotic cells (Kieke et al, 1999, Proc Natl Acad Sci USA, 96(10):5651-6) or may be expressed *in vitro* without involvement of cells (Hanes & Pluckthun, 1997, Proc Natl Acad Sci USA 94(10):4937-42; He & Taussig, 1997, Nucleic Acids Res 25(24):5132-4; Nemoto et al, 1997, FEBS Lett, 414(2):405-8).

In cases when protein-based libraries are used, the genes encoding the libraries of potential binding molecules are often packaged in viruses and the potential binding molecule displayed at the surface of the virus (Clackson *et al,* 1991, *supra;* Marks *et al,* 1991, *supra;* Smith, 1985, *supra*).

Perhaps the most commonly used display system is filamentous bacteriophage displaying antibody fragments at their surfaces, the antibody fragments being expressed as a fusion to the minor coat protein of the bacteriophage (Clackson *et al,* 1991, *supra;* Marks *et al,* 1991, *supra*). However, other suitable systems for display include using other viruses (EP 39578), bacteria (Gunneriusson *et al,* 1999, *supra;* Daugherty et al, 1998, Protein Eng 11(9):825-32; Daugherty et al, 1999, Protein Eng 12(7):613-21), and yeast (Shusta et al, 1999, J Mol Biol 292(5):949-56).

In addition, display systems have been developed utilising linkage of the polypeptide product to its encoding mRNA in so-called ribosome display systems (Hanes & Pluckthun, 1997, *supra;* He & Taussig, 1997, *supra;* Nemoto *et al,* 1997, *supra*), or alternatively linkage of the polypeptide product to the encoding DNA (see US Patent No. 5,856,090 and WO 98/37186).

The variable heavy ($V_H$) and variable light ($V_L$) domains of the antibody are involved in antigen recognition, a fact first recognised by early protease digestion experiments. Further confirmation was found by "humanisation" of rodent antibodies. Variable domains of rodent origin may be fused to constant domains of human origin such that the resultant antibody retains the antigenic specificity of the rodent parented antibody (Morrison et al (1984) Proc. Natl. Acad. Sci. USA 81, 6851-6855).

That antigenic specificity is conferred by variable domains and is independent of the constant domains is known from experiments involving the bacterial expression of antibody fragments, all containing one or more variable domains. These molecules include Fab-like molecules (Better et al (1988) Science 240, 1041); Fv molecules (Skerra et al (1988) Science 240, 1038); single-chain Fv (ScFv) molecules where the $V_H$ and $V_L$ partner domains are linked via a flexible oligopeptide (Bird et al (1988) Science 242, 423; Huston et al (1988) Proc. Natl. Acad. Sci. USA 85, 5879) and single domain antibodies (dAbs) comprising isolated V domains (Ward et al (1989) Nature 341, 544). A general review of the techniques involved in the synthesis of antibody fragments which retain their specific binding sites is to be found in Winter & Milstein (1991) Nature 349, 293-299.

The antibody or antigen-binding fragment may be selected from the group consisting of intact antibodies, Fv fragments (e.g. single chain Fv and disulphide-bonded Fv), Fab-like fragments (*e.g.* Fab fragments, Fab' fragments and F(ab)$_2$ fragments), single variable domains (*e.g.* $V_H$ and $V_L$ domains) and domain antibodies (dAbs, including single and dual formats [*i.e.* dAb-linker-dAb]). Preferably, the antibody or antigen-binding fragment is a single chain Fv (scFv).

The one or more binding moieties may alternatively comprise or consist of an antibody-like binding agent, for example an affibody or aptamer.

By "scFv molecules" we mean molecules wherein the $V_H$ and $V_L$ partner domains are linked via a flexible oligopeptide.

The advantages of using antibody fragments, rather than whole antibodies, are several-fold. The smaller size of the fragments may lead to improved pharmacological properties, such as better penetration of solid tissue. Effector functions of whole antibodies, such as complement binding, are removed. Fab, Fv, ScFv and dAb antibody fragments can all be expressed in and secreted from *E. coli,* thus allowing the facile production of large amounts of the said fragments.

Whole antibodies, and F(ab')$_2$ fragments are "bivalent". By "bivalent" we mean that the said antibodies and F(ab')$_2$ fragments have two antigen combining sites. In contrast, Fab, Fv, ScFv and dAb fragments are monovalent, having only one antigen combining sites.

The antibodies may be monoclonal or polyclonal. Suitable monoclonal antibodies may be prepared by known techniques, for example those disclosed in "Monoclonal Antibodies: A manual of techniques", H Zola (CRC Press, 1988) and in "Monoclonal Hybridoma Antibodies: Techniques and applications", J G R Hurrell (CRC Press, 1982).

When potential binding molecules are selected from libraries, one or more selector peptides having defined motifs are usually employed. Amino acid residues that provide structure, decreasing flexibility in the peptide or charged, polar or hydrophobic side chains allowing interaction with the binding molecule may be used in the design of motifs for selector peptides. For example:

(i) Proline may stabilise a peptide structure as its side chain is bound both to the alpha carbon as well as the nitrogen;
(ii) Phenylalanine, tyrosine and tryptophan have aromatic side chains and are highly hydrophobic, whereas leucine and isoleucine have aliphatic side chains and are also hydrophobic;
(iii) Lysine, arginine and histidine have basic side chains and will be positively charged at neutral pH, whereas aspartate and glutamate have acidic side chains and will be negatively charged at neutral pH;
(iv) Asparagine and glutamine are neutral at neutral pH but contain a amide group which may participate in hydrogen bonds;
(v) Serine, threonine and tyrosine side chains contain hydroxyl groups, which may participate in hydrogen bonds.

Typically, selection of binding molecules may involve the use of array technologies and systems to analyse binding to spots corresponding to types of binding molecules.

The one or more protein-binding moieties may comprise a detectable moiety. The detectable moiety may be selected from the group consisting of a fluorescent moiety, a luminescent moiety, a chemiluminescent moiety, a radioactive moiety and an enzymatic moiety.

In a further embodiment of the methods of the invention, step (c) may be performed using an assay comprising a second binding agent capable of binding to the one or more proteins, the second binding agent also comprising a detectable moiety. Suitable second binding agents are described in detail above in relation to the first binding agents.

Thus, the proteins of interest in the sample to be tested may first be isolated and/or immobilised using the first binding agent, after which the presence and/or relative amount of said biomarkers may be determined using a second binding agent.

In one embodiment, the second binding agent is an antibody or antigen-binding fragment thereof; typically a recombinant antibody or fragment thereof. Conveniently, the antibody or fragment thereof is selected from the group consisting of: scFv; Fab; a binding domain of an immunoglobulin molecule. Suitable antibodies and fragments, and methods for making the same, are described in detail above.

Alternatively, the second binding agent may be an antibody-like binding agent, such as an affibody or aptamer.

Alternatively, where the detectable moiety on the protein in the sample to be tested comprises or consists of a member of a specific binding pair (*e.g.* biotin), the second binding agent may comprise or consist of the complimentary member of the specific binding pair (*e.g.* streptavidin).

Where a detection assay is used, it is preferred that the detectable moiety is selected from the group consisting of: a fluorescent moiety; a luminescent moiety; a chemiluminescent moiety; a radioactive moiety; an enzymatic moiety. Examples of suitable detectable moieties for use in the methods of the invention are described above.

Preferred assays for detecting serum or plasma proteins include enzyme linked immunosorbent assays (ELISA), radioimmunoassay (RIA), immunoradiometric assays (IRMA) and immunoenzymatic assays (IEMA), including sandwich assays using monoclonal and/or polyclonal antibodies. Exemplary sandwich assays are described by David et al in US Patent Nos. 4,376,110 and 4,486,530. Antibody staining of cells on slides may be used in methods well known in cytology laboratory diagnostic tests, as well known to those skilled in the art.

Thus, in one embodiment the assay is an ELISA (Enzyme Linked Immunosorbent Assay) which typically involves the use of enzymes which give a coloured reaction product, usually in solid phase assays. Enzymes such as horseradish peroxidase and phosphatase have been widely employed. A way of amplifying the phosphatase reaction is to use NADP as a substrate to generate NAD which now acts as a coenzyme for a second enzyme system. Pyrophosphatase from *Escherichia coli* provides a good conjugate because the enzyme is not present in tissues, is stable and gives a good reaction colour. Chemiluminescent systems based on enzymes such as luciferase can also be used.

Conjugation with the vitamin biotin is frequently used since this can readily be detected by its reaction with enzyme-linked avidin or streptavidin to which it binds with great specificity and affinity.

In an alternative embodiment, the assay used for protein detection is conveniently a fluorometric assay. Thus, the detectable moiety of the second binding agent may be a fluorescent moiety, such as an *Alexa* fluorophore (for example Alexa-647).

Preferably, steps (c), (e), and/or (g) of the methods described in the first aspect are performed using an array. The array may be a bead-based array or a surface-based array. The array may be selected from the group consisting of: macroarray; microarray; nanoarray.

Arrays *per se* are well known in the art. Typically they are formed of a linear or two-dimensional structure having spaced apart (*i.e.* discrete) regions ("spots"), each having a finite area, formed on the surface of a solid support. An array can also be a bead structure where each bead can be identified by a molecular code or colour code or identified in a continuous flow. Analysis can also be performed sequentially where the sample is passed over a series of spots each adsorbing the class of molecules from the solution. The solid support is typically glass or a polymer, the most commonly used polymers being cellulose, polyacrylamide, nylon, polystyrene, polyvinyl chloride or polypropylene. The solid supports may be in the form of tubes, beads, discs, silicon chips, microplates, polyvinylidene difluoride (PVDF) membrane, nitrocellulose membrane, nylon membrane, other porous membrane, non-porous membrane (e.g. plastic, polymer, perspex, silicon, amongst others), a plurality of polymeric pins, or a plurality of microtitre wells, or any other surface suitable for immobilising proteins, polynucleotides and other suitable molecules and/or conducting an immunoassay. The binding processes are well known in the art and generally consist of cross-linking covalently binding or physically adsorbing a protein molecule, polynucleotide or the like to the solid support. Alternatively, affinity coupling of the probes via affinity-tags or similar constructs may be employed. By using well-known techniques, such as contact or non-contact printing, masking or photolithography, the location of each spot can be defined. For reviews see Jenkins, R.E., Pennington, S.R. (2001, Proteomics, 2,13-29) and Lal et al (2002, Drug Discov Today 15;7(18 Suppl):S143-9).

Typically the array is a microarray. By "microarray" we include the meaning of an array of regions having a density of discrete regions of at least about $100/cm^2$, and preferably at least about $1000/cm^2$. The regions in a microarray have typical dimensions, e.g. diameter, in the range of between about 10-250 $\mu m$, and are separated from other regions in the array by about the same distance. The array may alternatively be a macroarray or a nanoarray.

Once suitable binding molecules (discussed above) have been identified and isolated, the skilled person can manufacture an array using methods well known in the art of molecular biology.

In an additional or alternative embodiment one or more biomarkers measured in step (c) comprise or consist of one or more homologous gene product expressed by human cells. In an additional or alternative embodiment one or more biomarkers measured in step (c) is a protein or polypeptide. In an additional or alternative embodiment one or more biomarker measured in step (c) is a nucleic acid (e.g., DNA, mRNA or cDNA etc).

The method of claim 1 is performed in vitro. In a further disclosed embodiment, which is not part of the invention, the method is performed *in vivo, ex vivo* or *in silico*.

By "test agent" we include any substance, compound, composition, and/or entity (or mixture thereof) for which respiratory sensitization status is to be determined.

By "sensitization status" we include or mean whether or not a test agent (or mixture of test agent) is a sensitizer or not (e.g., a respiratory sensitizer).

In one embodiment, the method is for identifying agents capable of inducing a respiratory hypersensitivity response. Preferably, the hypersensitivity response is a humoral hypersensitivity response, for example, a type I hypersensitivity response. In one embodiment, the method is for identifying agents capable of inducing respiratory allergy.

By "indicative of the respiratory sensitizing effect of the test agent" we include determining whether or not the test agent is a respiratory sensitizer and/or determining the potency of the test agent as a respiratory sensitizer.

By agents "capable of inducing respiratory sensitization" we mean any agent capable of inducing and triggering a Type I immediate hypersensitivity reaction in the respiratory tract of a mammal. Preferably the mammal is a human. Preferably, the Type I immediate hypersensitivity reaction is DC-mediated and/or involves the differentiation of T cells into Th2 cells. Preferably the Type I immediate hypersensitivity reaction results in humoral immunity and/or respiratory allergy.

The conducting zone of the mammalian lung contains the trachea, the bronchi, the bronchioles, and the terminal bronchioles. The respiratory zone contains the respiratory bronchioles, the alveolar ducts, and the alveoli. The conducting zone is made up of airways, has no gas exchange with the blood, and is reinforced with cartilage in order to hold open the airways. The conducting zone humidifies inhaled air and warms it to 37°C (99°F). It also cleanses the air by removing particles via cilia located on the walls of all the passageways. The respiratory zone is the site of gas exchange with blood.

In one embodiment, the agent "capable of inducing respiratory sensitization" is an agent capable of inducing and triggering a Type I immediate hypersensitivity reaction at a site of lung epithelium in a mammal. Preferably, the site of lung epithelium is in the respiratory zone of the lung, but may alternatively or additionally be in the conductive zone of the lung.

The mammal may be any domestic or farm animal. Preferably, the mammal is a rat, mouse, guinea pig, cat, dog, horse or a primate. Most preferably, the mammal is human.

Dendritic cells (DCs) are immune cells forming part of the mammalian immune system. Their main function is to process antigen material and present it on the surface to other cells of the immune system (i.e., they function as antigen-presenting cells), bridging the innate and adaptive immune systems.

Dendritic cells are present in tissues in contact with the external environment, such as the skin (where there is a

13

specialized dendritic cell type called Langerhans cells) and the inner lining of the nose, lungs, stomach and intestines. They can also be found in an immature state in the blood. Once activated, they migrate to the lymph nodes where they interact with T cells and B cells to initiate and shape the adaptive immune response. At certain development stages they grow branched projections, the dendrites. While similar in appearance, these are distinct structures from the dendrites of neurons. Immature dendritic cells are also called veiled cells, as they possess large cytoplasmic 'veils' rather than dendrites.

By "dendritic-like cells" we mean non-dendritic cells that exhibit functional and phenotypic characteristics specific to dendritic cells such as morphological characteristics, expression of costimulatory molecules and MHC class II molecules, and the ability to pinocytose macromolecules and to activate resting T cells.

In an additional or alternative embodiment the population of dendritic cells or population of dendritic-like cells comprises or consists of immortal cells. By "immortal" we mean cells that are not limited by a point at which they can no longer continue to divide, which might otherwise be due to DNA damage or shortened telomeres.

In an additional or alternative embodiment the population of dendritic cells or population of dendritic-like cells comprises or consists of non-naturally occurring cells. By "non-naturally occurring" cells, we mean that the cells are different to, modified from, or variants of, those which would be found in nature; in other words, they are not cells which would normally occur in nature. For example, the cells are different to, modified from, and/or a variant of, a naturally occurring human myeloid leukaemia cell or a naturally occurring dendritic cell.

In an additional or alternative embodiment the population of dendritic cells or population of dendritic-like cells is a population of dendritic-like cells. In an additional or alternative embodiment the dendritic-like cells are myeloid dendritic-like cells. In an additional or alternative embodiment the myeloid dendritic-like cells are derived from myeloid dendritic cells. In an additional or alternative embodiment the cells derived from myeloid dendritic cells are myeloid leukaemia-derived cells. In an additional or alternative embodiment the myeloid leukaemia-derived cells are selected from the group consisting of KG-1, THP-1, U-937, HL-60, Monomac-6, AML-193, MUTZ-3, and SenzaCell.

In an additional or alternative embodiment the dendritic-like cells are MUTZ-3 cells. MUTZ-3 cells are human acute myelomonocytic leukemia cells that are available from Deutsche Sammlung für Mikroorganismen und Zellkulturen GmbH (DSMZ), Braunschweig, Germany (www.dsmz.de; DMSZ No. ACC 295).

In an additional or alternative embodiment the dendritic-like cells are non-naturally occurring dendritic-like myeloid leukaemia cells according to ATCC Patent Deposit Designation PTA-123875. These cells are also referred to as "SenzaCell". SenzaCell (ATCC Patent Deposit Designation PTA-123875) is deposited at the American Type Culture Collection (ATCC), 10801 University Blvd, Manassas, VA 20110, USA.

In an additional or alternative embodiment the myeloid leukaemia-derived cells are MUTZ-3 or SenzaCell.

In one embodiment, the dendritic-like cells, after stimulation with cytokine, present antigens through CD1d, MHC class I and II and/or induce specific T-cell proliferation.

In one embodiment, the dendritic-like cells are CD34$^+$ dendritic cell progenitors. Optionally, the CD34$^+$ dendritic cell progenitors can acquire, upon cytokine stimulation, the phenotypes of presenting antigens through CD1d, MHC class I and II, induce specific T-cell proliferation, and/or displaying a mature transcriptional and phenotypic profile upon stimulation with inflammatory mediators (i.e. similar phenotypes to immature dendritic cells or Langerhans-like dendritic cells).

In one embodiment, the dendritic-like cells express at least one of the markers selected from the group consisting of CD54, CD86, CD80, HLA-DR, CD14, CD34 and CD1a, for example, 2, 3, 4, 5, 6 or 7 of the markers. In a further embodiment, the dendritic-like cells express the markers CD54, CD86, CD80, HLA-DR, CD14, CD34 and CD1a.

In one embodiment, the population of dendritic cells or population of dendritic-like cells is a population of dendritic cells. Preferably, the dendritic cells are primary dendritic cells. Preferably, the dendritic cells are myeloid dendritic cells.

Dendritic cells may be recognized by function, by phenotype and/or by gene expression pattern, particularly by cell surface phenotype. These cells are characterized by their distinctive morphology, high levels of surface MHC-class II expression and ability to present antigen to CD4+ and/or CD8+ T cells, particularly to naïve T cells (Steinman et al. (1991) Ann. Rev. Immunol. 9: 271).

The cell surface of dendritic cells is unusual, with characteristic veil-like projections, and is characterized by expression of the cell surface markers CD11c and MHC class II. Most DCs are negative for markers of other leukocyte lineages, including T cells, B cells, monocytes/macrophages, and granulocytes. Subpopulations of dendritic cells may also express additional markers including 33D1, CCR1, CCR2, CCR4, CCR5, CCR6, CCR7, CD1a-d, CD4, CD5, CD8alpha, CD9, CD11b, CD24, CD40, CD48, CD54, CD58, CD80, CD83, CD86, CD91, CD117, CD123 (IL3Ra), CD134, CD137, CD150, CD153, CD162, CXCR1, CXCR2, CXCR4, DCIR, DC-LAMP, DC-SIGN, DEC205, E-cadherin, Langerin, Mannose receptor, MARCO, TLR2, TLR3 TLR4, TLR5, TLR6, TLR9, and several lectins.

The patterns of expression of these cell surface markers may vary along with the maturity of the dendritic cells, their tissue of origin, and/or their species of origin. Immature dendritic cells express low levels of MHC class II, but are capable of endocytosing antigenic proteins and processing them for presentation in a complex with MHC class II molecules. Activated dendritic cells express high levels of MHC class 11, ICAM-1 and CD86, and are capable of stimulating the proliferation of naive allogeneic T cells, e. g. in a mixed leukocyte reaction (MLR).

Functionally, dendritic cells or dendritic-like cells may be identified by any convenient assay for determination of antigen presentation. Such assays may include testing the ability to stimulate antigen- primed and/or naive T cells by presentation of a test antigen, followed by determination of T cell proliferation, release of IL-2, and the like.

In one embodiment the dendritic-like cells include epithelial cells and/or epithelial-like cells such as BEAS-2B [28], WT 9-7 and A549[29]. Preferably the epithelial cells are lung epithelial cells. Preferably the epithelial-like cells are lung epithelial-like cells. In an alternative embodiment the dendritic-like cells include epithelial cells and/or epithelial-like cells.

Methods of detecting and/or measuring the concentration of protein and/or nucleic acid are well known to those skilled in the art, see for example Sambrook and Russell, 2001, Cold Spring Harbor Laboratory Press.

Preferred methods for detection and/or measurement of protein include Western blot, North-Western blot, immunosorbent assays (ELISA), antibody microarray, tissue microarray (TMA), immunoprecipitation, *in situ* hybridisation and other immunohistochemistry techniques, radioimmunoassay (RIA), immunoradiometric assays (IRMA) and immunoenzymatic assays (IEMA), including sandwich assays using monoclonal and/or polyclonal antibodies. Exemplary sandwich assays are described by David et al., in US Patent Nos. 4,376,110 and 4,486,530. Antibody staining of cells on slides may be used in methods well known in cytology laboratory diagnostic tests, as well known to those skilled in the art.

Typically, ELISA involves the use of enzymes which give a coloured reaction product, usually in solid phase assays. Enzymes such as horseradish peroxidase and phosphatase have been widely employed. A way of amplifying the phosphatase reaction is to use NADP as a substrate to generate NAD which now acts as a coenzyme for a second enzyme system. Pyrophosphatase from *Escherichia coli* provides a good conjugate because the enzyme is not present in tissues, is stable and gives a good reaction colour. Chemi-luminescent systems based on enzymes such as luciferase can also be used.

Conjugation with the vitamin biotin is frequently used since this can readily be detected by its reaction with enzyme-linked avidin or streptavidin to which it binds with great specificity and affinity.

In an additional or alternative embodiment, the method comprises one or more of the following steps:

(i) cultivating dendritic or dendritic-like cells;
(ii) seeding cells of (i) in one or more wells, preferably at steady state growth phase, e.g. wells of one or more multi-well assay plate;
(iii) adding to one or more well(s) of (ii) the agent(s) to be tested;
(iv) adding to one or more separate well(s) of (ii) positive control(s), e.g. Reactive Orange 16, Piperazine, Chloramine T and/or Trimellitic Anhydride;
(v) adding to one or more separate well(s) of (ii) negative control(s), e.g. DMSO; and/or leaving one or more separate well(s) of (ii) unstimulated to obtain a medium control and/or for normalization purposes;
(vi) incubating cells in wells of (iii)-(v), preferably for about 24 hours; and, optionally, harvesting cells from wells of (iii)-(v); and, further optionally, removing supernatant and storing in TRIzol reagent;
(vii) isolating purified total RNA from the cells of (vi) and, optionally, converting mRNA into cDNA;
(viii) quantifying expression levels of individual mRNA transcripts from (vii), e.g. using an array, such as an Affymetrix Human Gene 1.0 ST array, or using customized gene expression analysis probes, such as a Nanostring code set;
(ix) exporting and normalizing data from (viii), e.g. using appropriate algorithms such as is described in Table 4;
(x) isolating data from (ix) originating from biomarkers of the GARD Respiratory Prediction Signature (i.e. the biomarkers of Table A);
(xi) applying a prediction model to the data of (x), e.g. a frozen SVM model previously established and trained on historical data, e.g. data obtained in Example 1, see also coding in Table 4, to predict the respiratory sensitization status of tested agents(s) and negative/positive control(s);
(xii) identifying if the tested agent is an agent capable of inducing respiratory sensitization in a mammal.

A second aspect of the invention provides an array for use in the method according to the first aspect of the invention, the array consisting of binding moieties for all biomarkers listed in Table A as defined in the first aspect of the invention.

Also disclosed herein the array comprises one or more binding moiety for each of the biomarkers as defined in the first aspect of the invention. In an additional or alternative embodiment the one or more binding moiety is immobilised.

In an additional or alternative embodiment the array is a bead-based array. In an additional or alternative embodiment the array is a surface-based array. In an additional or alternative embodiment the array is selected from the group consisting of: macroarray; microarray; nanoarray.

Also disclosed herein, the array of the second aspect of the invention may comprise one or more, preferably two or more, binding moieties, wherein the binding moieties are each capable of binding selectively to a biomarker as defined in the first aspect. Therefore, also disclosed herein the array may comprise or consist of a particular selection of biomarker-specific binding moieties which correlates to any particular selection of biomarkers as defined in the first aspect.

For example, also disclosed herein, the array comprises 2, 3, 4, 5, 6, 7, 8, 9, 10, 11, 12, 13, 14, 15, 16, 17, 18, 19, 20, 21, 22, 23, 24, 25, 26, 27, or 28 different binding moieties, wherein the different binding moieties are each capable of binding selectively to a different biomarker listed in Table A. For example, the array may comprise or consist of 28 different binding moieties, each capable of binding selectively to a different biomarker listed in Table A. Also disclosed herein, the array comprises 2, 3, 4, 5, 6, 7, 8, 9, 10, 11, 12, 13, 14, 15, 16, 17, 18, 19, 20, 21, 22, 23, 24, or 25 different binding moieties, wherein the different binding moieties are each capable of binding selectively to a different biomarker listed in Table A(i). For example, also disclosed herein, the array may comprise or consist of 25 different binding moieties, each capable of binding selectively to a different biomarker listed in Table A(i).

A third aspect of the invention provides the use of the biomarkers as defined in the first aspect of the invention for determining the respiratory sensitizing effect of a test agent.

In an additional or alternative embodiment there is provided the use of the biomarkers from the group defined in Table A for determining the respiratory sensitizing effect of a test agent.

In an additional or alternative embodiment there is provided the use of binding moieties with specificity for each biomarker listed in Table A for determining the respiratory sensitizing effect of a test agent.

A fourth aspect of the invention provides an analytical kit for use in a method according the first aspect of the invention comprising:

(a) an array according to the second aspect of the invention; and

(b) instructions for performing the method as defined in the first aspect of the invention (optional).

In an additional or alternative embodiment the analytical kit further comprising one or more control agents as defined in the first aspect of the invention.

Also disclosed herein is a method of treating or preventing a respiratory type I hypersensitivity reaction (such as respiratory asthma) in a patient comprising the steps of:

(a) providing one or more test agent that the patient is or has been exposed to;
(b) determining whether the one or more test agent provided in step (a) is a respiratory sensitizer using a method provided in a first aspect of the invention; and
(c) where one or more test agent is identified as a respiratory sensitizer, reducing or preventing exposure of the patient to the one or more test agents and/or providing appropriate treatment for the symptoms of sensitization.

Preferably, the one or more test agent that the patient is or has been exposed to is an agent that the patient is presently exposed to at least once a month, for example, at least once every two weeks, at least once every week, or at least once every day.

Treatments of the symptoms of sensitization may include short-acting beta2-adrenoceptor agonists (SABA), such as salbutamol; anticholinergic medications, such as ipratropium bromide; other adrenergic agonists, such as inhaled epinephrine; Corticosteroids such as beclomethasone; long-acting beta-adrenoceptor agonists (LABA) such as salmeterol and formoterol; leukotriene antagonists such as montelukast and zafirlukast; and/or mast cell stabilizers (such as cromolyn sodium) are another non-preferred alternative to corticosteroids.

Preferably, the method of treatment is consistent with the method described in the first aspect of the invention, and one or more of the embodiments described therein.

Also disclosed herein is a computer program for operating the methods the invention, for example, for interpreting the expression data of step (c) (and subsequent expression measurement steps) and thereby determining whether one or more test agent is allergenic. The computer program may be a programmed SVM. The computer program may be recorded on a suitable computer-readable carrier known to persons skilled in the art. Suitable computer-readable-carriers may include compact discs (including CD-ROMs, DVDs, Blue Rays and the like), floppy discs, flash memory drives, ROM or hard disc drives. The computer program may be installed on a computer suitable for executing the computer program.

The skilled person will appreciate that all non-conflicting embodiments may be used in combination. Hence, embodiments from one aspect of the invention may equally be applied to a second aspect of the invention. The listing or discussion of an apparently prior-published document in the specification should not necessarily be taken as an acknowledgment that the document is part of the state of the art or is common general knowledge.

Preferred, non-limiting examples which embody certain aspects of the invention will now be described, with reference to the following figures:

**Figure 1. PCA of training data set in a compressed space of 28 variables, originating from an optimized biomarker signature.**

**Figure 2. Visualization of classification results of test set 1, using the finalized GARDair prediction model.**
A test substance is classified as a respiratory sensitizer if the mean SVM decision value (n=3) is greater than 0.

**Figure 3. Visualization of classification results of test set 2, using the finalized GARDair prediction model.**
A test substance is classified as a respiratory sensitizer if the mean SVM decision value (n=3) is greater than 0.

**EXAMPLE 1**

Results

*Prediction Model Rationale*

GARD™ is a state-of-the-art methodology platform for assessment of chemical sensitizers. It is based on a dendritic cell (DC)-like cell line, thus mimicking the cell type involved in the initiation of the response leading to sensitization. Cultivated DCs are exposed to test substances of interest. Following incubation, exposure-induced transcriptional changes are measured in order to study the activation state of the cells. These changes are associated with the bridging of innate and adaptive immune responses and the decision-making role of DCs in vivo and constitutes of e.g. up-regulation of co-stimulatory molecules, induction of cellular and oxidative stress pathways and an altered phenotype associated with migratory and inter-cell communication functions. By using state-of-the-art gene expression technologies, high informational content data is generated, that allows the user to get a holistic view of the cellular response induced by the test substance. Simplified, the described technology allows the assessment of the test substance as a sensitizer or a non-sensitizer.
GARD is considered a testing strategy platform, on which is based a number of applications. The term "platform" here indicates that all applications are based on the same experimental strategy and similar experimental protocols. The term "application" here indicates different assays for different biological endpoints.
The "GARDair" assay described herein is a novel assay based on the GARD platform that here is demonstrated to have the capacity to accurately classify respiratory sensitizers. Thus, GARDair has the capacity to be the preferred test method that specifically classifies chemicals as respiratory sensitizers, an endpoint to which validated, or even widely accepted and used, prediction models currently do not exist.

*GARDair biomarker discovery*

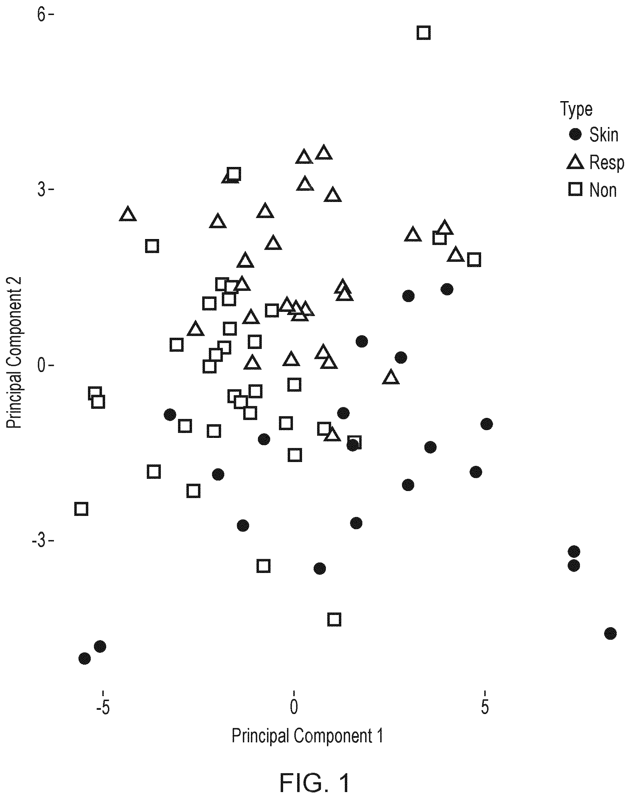

SenzaCells (ATCC Deposit #PTA-123875) were exposed to a reference panel of chemicals, comprising 10 well characterized respiratory sensitizers and 20 non-respiratory sensitizers, as defined by available literature and expert consensus (Chan-Yeung & Malo, 1994, Dearman et al., 1997, Dearman et al., 2012, Lalko et al., 2012). Of note, the set of non-respiratory sensitizers include skin sensitizers without any recorded capability to induce respiratory sensitization. This set of reference chemicals were used to create what is typically referred to as a training data set, and it is listed in Table 1. All exposures were performed in repeated triplicate experiments in a controlled setting, thus generating a coherent dataset with high statistical power optimized for subsequent biomarker discovery.
Purified RNA from chemically exposed cell cultures were isolated and gene expression analysis was performed using Affymetrix microarrays, thus generating a whole genome expression data set for information mining, referred to as the training data set. The statistical power of the training data set was further increased by the application of a Surrogate Variable Analysis (SVA) algorithm, which identifies and subsequently eliminates noise signals originating from surrogate variables that are statistically unrelated to the biological endpoint of interest. Next, analysis of variance (ANOVA) was applied to identify differentially expressed genes (DEGs). Using an adjusted p-value (i.e. the q-value, a p-value corrected for multiple hypothesis testing using the Benjamini-Hochberg method) of <0.05 as a definition of statistical significance, 28 DEGS met the selection criteria. The identities of the 28 DEGs, henceforth collectively referred to as the GARD respiratory prediction signature (GRPS), are presented in Table 2. Furthermore, the training data set is visualized using principal component analysis (PCA) in Figure 1.
The respective weightings of the 28 genes in the SVM model are indicated in Table 5. The SVM is an algorithm that defines a prediction model. Once the model is defined (i.e. trained) the actual prediction model can be represented by a linear equation, as so:

$$DV = K1*X2 + K2*X3 + … + KN*XN + M$$

In which DV is the decision value (the output of the model when applied), Ks are constants, Xs are independent variables and M is a constant representing an intercept. In this case, N is 28. Expression levels of 28 genes (i.e. Xs) were measured and a defined equation used with 28 fixed Ks and M to calculate DV.

The weights provided are the Ks, i.e. the constant with which each gene expression level is multiplied. Thus, the bigger the K, the more impact the corresponding gene X will have on the DV. As a simplified example, consider the case in which N=1. This will give the commonly known equation for a straight line, i.e. Y = KX + M.

*Technology platform transfer and prediction model definition*

Following the establishment of the GRPS, hybridizing probes were designed for standardized measurements of the GRPS using the Nanostring nCounter system (Geiss et al., 2008). This work was performed in a close analogy of the technology transfer of GARDskin, progress which has been previously published (Forreryd et al., 2016). Utilizing identical cellular protocols as the afore-mentioned assay facilitates a robust, simple and resource-effective assay. A prediction model was trained and frozen, based on a Support Vector Machine (SVM), using the samples of the training data set with a binary "function in study" (respiratory sensitizer / non-respiratory sensitizer) as the dependent variable, and the gene expression values of the GRPS as the independent variables (i.e. predictors), see also Table 4.

*Proof of concept - classifications of external test data.*

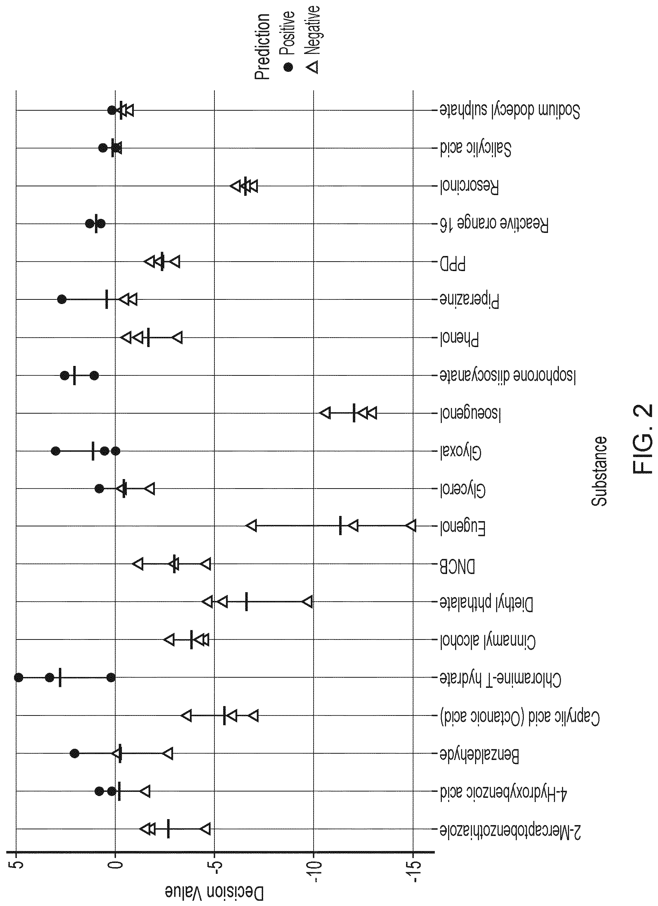

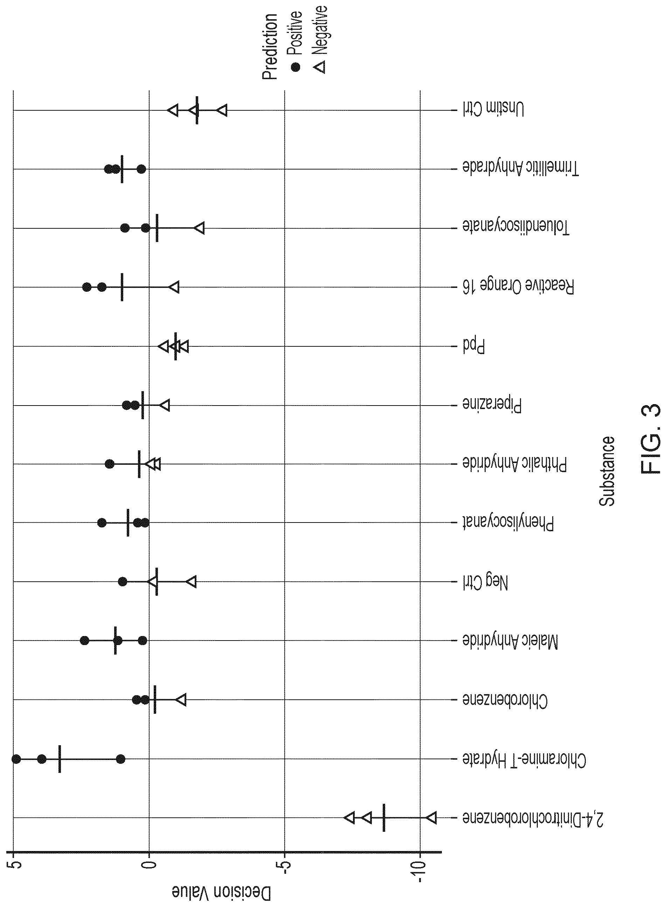

Having established an optimized prediction model and associated protocols, the assay was challenged with two sets of external samples, referred to as test data sets. The chemical identities of included samples in the test sets, their true group belonging (respiratory sensitizers or non-respiratory sensitizers) and the GARDair classification results are listed in Table 3. Graphical representations of classifications, as defined by generated GARDair decision values, are shown in Figures 2 and 3, for test sets 1 and 2, respectively.

Estimating the predictive performance of GARDair based on the available data, the predictive accuracy was calculated to 89%, well-balanced between sensitivity and specificity. Furthermore, based on the few repeated exposures available from independent experiments, the reproducibility was 100%, indicative of a robust assay.

Discussion

Based on the here within presented data, it was concluded that the concept of utilizing the GARD platform, e.g. exposing DC-like cells to test substances and interrogating the induced transcriptional pattern for machine-learning assisted classification is a functional strategy for assessment of chemical respiratory sensitizers.

GARDair is to date a finalized assay, based on a genomic readout, as measured by a state-of-the-art platform, of chemically exposed DC-like cells in vitro. The assay has been demonstrated to be functional and robust. The assay is proposed to monitor transcriptional changes in DCs, as induced specifically by respiratory sensitizers, related to the bridging of innate and adaptive immune functions and skewing towards Th2 type immune responses. Primarily, this is demonstrated by the data-driven identification of IL7R and CRLF2 genes, which as translated proteins together form the receptor for thymoid stromal lymphopoietin (TSLP). TSLP ligand-binding to the TSLP receptor of antigen presenting cells has been previously shown to drive Th2 differentiation (Paul & Zhu, 2010, Soumelis et al., 2002). However, it has previously not been described in relation to induction of respiratory sensitization to chemicals.

Material & Methods

*Cell line maintenance and seeding of cells for stimulation*

The human myeloid leukemia-derived cell line SenzaCell (available through ATCC), acting as an in vitro model of human Dendritic Cell (DC), is maintained in $\alpha$-MEM (Thermo Scientific Hyclone, Logan, UT) supplemented with 20% (volume/volume) fetal calf serum (Life Technologies, Carlsbad, CA) and 40 ng/ml recombinant human Granulocyte Macrophage Colony Stimulating Factor (rhGM-CSF) (Miltenyi Biotec, Germany). A media change during expansion is performed every 3-4 days. Working stocks of cultures are grown for a maximum of 16 passages or two months after thawing. For chemical stimulation of cells, exposed cells are incubated for 24h at 37°C, 5% $CO_2$ and 95% humidity.

*Test Substance handling and assessment of cytotoxicity*

All Test Substances were stored according to instructions from the supplier, to ensure stability of Test Substances. Test Substances were dissolved in DMSO or water, based on physical properties. As many Test Substances will have a toxic effect on the cells, cytotoxic effects of Test Substances were monitored. Some Test Substances were poorly dissolved in cell media; therefore, the maximum soluble concentration was assessed as well. The Test Substance that was to be tested was titrated to concentrations ranging from 1 $\mu$M to the maximum soluble concentration in cell media. For freely soluble Test Substances, 500 $\mu$M was set as the upper limit of the titration range. For Test Substances dissolved in DMSO, the in-well concentration of DMSO was 0.1%. After incubation for 24 h at 37°C, 5% CO2 and 95% humidity, harvested cells were stained with the viability marker Propidium Iodide (PI) (BD Bioscience, USA) and analyzed by flow cytometry. PI-negative cells were defined as viable, and the relative viability of cells stimulated with each concentration in the titration range was calculated as

$$\text{Relative viability} = (\text{fraction of viable stimulated cells})/(\text{fraction of viable unstimulated cells}) \cdot 100$$

For toxic Test Substances, the concentration yielding 90% relative viability (Rv90) was used for the GARD assay, the reason being that this concentration demonstrates bioavailability of the Test Substance used for stimulation, while not impairing immunological responses. For non-toxic Test Substances, a concentration of 500 $\mu$M was used if possible. For non-toxic Test Substances that were insoluble at 500 $\mu$M in cell media, the highest soluble concentration was used. Whichever of these three criteria was met, only one concentration will be used for gene expression analysis. The concentration to be used for any given chemical was termed the 'GARD input concentration'.

*GARD Main Stimulation*

Once the GARD input concentration for Test Substances to be assayed was established, the cells were stimulated again as described above, this time only using the GARD input concentration. All assessments of Test Substances and Benchmark Controls were assayed in biological triplicates, performed at different time-points and using different cell cultures. After incubation for 24 h at 37°C, 5% CO2 and 95% humidity, cell culture was lysed in TRIzol reagent (Life Technologies) and stored at -20°C until RNA was extracted. In parallel, a small sample of stimulated cells was taken for PI staining and analysis with flow cytometry, to ensure the expected relative viability of stimulated cells was reached.

*Isolation of RNA*

RNA isolation from lysed cells was performed using commercially available kits (Direct-Zol RNA MiniPrep, Zymo Research, Irvine, CA). Total RNA was quantified and quality controlled using BioAnalyzer equipment (Agilent, Santa Clara, CA).

*Gene expression analysis using microarrays*

Preparation of cDNA and hybridization to HuGene ST 1.0 microarrays were performed by Swegene Centre for Integrative Biology at Lund University (SCIBLU, Lund, Sweden), according to the manufacturer's recommended protocols, kits and reagents (Affymetrix, Santa Clara, CA).

*Microarray data acquisition and normalization*

Hybridized microarrays were washed and scanned according to recommended protocols. Raw data .cell-files were imported into the R environment for statistical computing (www.r-project.org). Raw data were normalized and converted to gene expression signals using the R-package SCAN.

*Data analysis - Feature selection of GARDair sensitization biomarker signature*

Normalized data containing biological triplicates of SenzaCell samples stimulated with the panel of chemicals listed in Table 1 were mined for differentially regulated genes, able to discriminate between respiratory sensitizers and respiratory non-sensitizers. Unwanted variation from undefined sources was removed using Surrogate Variable Analysis, available from the R-package SVA. Regulated genes were identified using an ANOVA from the R-package Limma. Genes with a false discovery rate (i.e. the q-value, a p-value corrected for multiple hypothesis testing using the Benjamini-Hochberg method) $<0.05$ were considered statistically significant. 28 unique genes met the selection criteria and they are presented in Table 2.

*Technology platform transfer*

Unique Nanostring nCounter system transcript probes were synthesized by the Nanostring Bioinformatics team (Nanostring, Seattle, WA). Following protocols by the supplier (Nanostring), Nanostring gene expression data was generated from the RNA samples produced for biomarker discovery, i.e. a complete reproduction of the training data set (Table 1), covering the 28 genes of interest.

*Prediction model establishment and testing of external test chemicals*

A Support Vector Machine (SVM) was trained on Nanostring expression data generated by the training data set (Table 1), using the "Function in study" as dependent variable (i.e. parameter to be predicted) and the 28 genes of the biomarker signature as independent variables (i.e. predictors), using the R statistical environment (R Core Team) and additional packages (see Table 4). For testing of external test chemicals, gene expression data was generated according to protocols described above. The trained SVM model was applied to classify each sample as respiratory sensitizer or non-respiratory sensitizer, as determined by the mean SVM decision value (n=3). Positive decision values denotes a positive classification.

**References**

Chan-Yeung & Malo, 1994. Aetiological agents in occupational asthma. European Respiratory Journal.

Dearman et al., 1997. Classification of chemical allergens according to cytokine secretion profiles of murine local lymph node cells. Journal of Applied Toxicology.

Dearman et al., 2011. Inter-relationships between different classes of chemical allergens. Journal of Applied Toxicology.

Dearman et al., 2012. Inter-relationships between different classes of chemical allergens. Journal of Applied Toxicology.

Forreryd et al., 2015. Prediction of chemical Respiratory sensitizers using GARD, a novel in vitro assay based on a genomic biomarker signature. PLoS One 10(3).

Forreryd et al., 2016. From genome-wide arrays to tailor-made biomarker readout - Progress towards routine analysis of skin sensitizing chemicals with GARD. Toxicology in vitro.

Geiss et al., 2008. Direct multiplexed measurement of gene expression with color-coded probe pairs. Nature Biotechnology.

Isola et al., 2008. Chemical respiratory allergy and occupational asthma: what are the key areas of uncertainty? Journal of Applied Toxicology.

Johansson et al., 2011. A genomic biomarker signature can predict skin sensitizers using a cell-based in vitro alternative to animal tests. BMC Genomics.

Kimber et al., 2002. Chemical respiratory allergy: role of IgE antibody and relevance of route of exposure. Toxicology.

Kimber et al., 2011. Chemical allergy: translating biology into hazard characterization. Toxicological Sciences.

Kimber et al., 2014. Chemical respiratory allergy: reverse engineering an adverse outcome pathway. Toxicology.

Lalko et al., 2012. The direct peptide reactivity assay: selectivity of chemical respiratory allergens. Toxicological Sciences.

Paul & Zhu, 2010. How are Th2-type immune responses initiated and amplified. Nature Reviews Immunology.

Soumelis et al., 2002. Human epithelial cells trigger dendritic cell-mediated allergic inflammation by producing TSLP. Nat Immunol.

Sullivan et al., 2017. An Adverse Outcome Pathway for Sensitization of the Respiratory Tract by Low-Molecular-Weight Chemicals: Building Evidence to Support the Utility of In Vitro and In Silico Methods in a Regulatory Context. Applied in vitro Toxicology.

**Tables**

**Table A**

| Gene name | Gene Symbol | Entrez ID | Affymetrix ID | Weight |
|---|---|---|---|---|
| **Table A(i)** | | | | |
| *cytokine receptor-like factor 2* | CRLF2 | 64109 | 8171105 | -1.01835933608703 |
| *fascin actin-bundling protein 1* | FSCN1 | 6624 | 8131339 | 1.00203258207129 |
| *amino-terminal enhancer of split* | AES | 166 | 8032576 | -0.937232228971051 |

(continued)

| Gene name | Gene Symbol | Entrez ID | Affymetrix ID | Weight |
|---|---|---|---|---|
| **Table A(i)** | | | | |
| arachidonate 5-lipoxygenase activating protein | ALOX5AP | 241 | 7968344 | 0.859616973865753 |
| RAB27B, member RAS oncogene family | RAB27B | 5874 | 8021301 | 0.782688844360711 |
| ZFP36 ring finger protein like 1 | ZFP36L1 | 677 | 7979813 | -0.719233666771149 |
| solute carrier family 44 member 2 | SLC44A2 | 57153 | 8025672 | 0.718226173217911 |
| atlastin GTPase 1 | ATL1 | 51062 | 7974270 | 0.699374841646448 |
| family with sequence similarity 30 member A | FAM30A | 9834 | 7977440 | 0.683461721920966 |
| cathepsin H | CTSH | 1512 | 7990757 | -0.65487992465195 |
| ninjurin 1 | NINJ1 | 4814 | 8162455 | -0.577359642405239 |
| Ral GTPase activating protein catalytic alpha subunit 2 | RALGAPA2 | 57186 | 8065280 | 0.552163931377946 |
| ring finger protein 220 | RNF220 | 55182 | 7900979 | -0.551522449893945 |
| oxysterol binding protein like 3 | OSBPL3 | 26031 | 8138613 | -0.538467358395433 |
| calcium voltage-gated channel auxiliary subunit alpha2delta 2 | CACNA2D2 | 9254 | 8087691 | -0.51849673058401 |
| Heterogeneous Nuclear Ribonucleoprotein C (C1/C2) | HNRNPC | 3183 | 7893129 | 0.299399629874934 |
| phosphatidylinositol 3-kinase catalytic subunit type 3 | PIK3C3 | 5289 | 8021015 | -0.256425970684912 |
| HOP homeobox | HOPX | 84525 | 8100507 | 0.166534308063369 |
| versican | VCAN | 1462 | 8106743 | -0.147007737618858 |
| RUN and FYVE domain containing 1 | RUFY1 | 80230 | 8110499 | 0.0996656054685292 |
| G protein subunit alpha 15 | GNA15 | 2769 | 8024572 | 0.0794276641913698 |
| ADAM metallopeptidase domain 8 | ADAM8 | 101 | 7937150 | -0.0746172327492091 |
| nuclear receptor interacting protein 1 | NRIP1 | 8204 | 8069553 | 0.0715765479932369 |
| CCCTC-binding factor | CTCF | 10664 | 7996593 | 0.0477003538478608 |
| phosphatidylinositol specific phospholipase C X domain containing 1 | PLCXD1 | 55344 | 8165711 | 0.0263482446344047 |
| **Table A(ii)** | | | | |
| MYCN proto-oncogene, bHLH transcription factor | MYCN | 4613 | 8040419 | -0.775008003430203 |
| interleukin 7 receptor | IL7R | 3575 | 8104901 | 0.215964226173642 |
| RAS like proto-oncogene A | RALA | 5898 | 8132406 | -0.101979863027782 |

**Table 1.** Chemical constituents of the training data set

| Chemical Name | CAS | Function in study |
|---|---|---|
| ammonium hexachloroplatinate | 16919-58-7 | RS |
| ammonium persulfate | 7727-54-0 | RS |
| ethylendiamine | 107-15-3 | RS / SS |
| glutaraldehyde | 111-30-8 | RS |

(continued)

| Chemical Name | CAS | Function in study |
|---|---|---|
| hexamethylen diisocyanate | 822-06-0 | RS |
| maleic anhydride | 108-31-6 | RS |
| methylene diphenol diisocyanate | 101-68-8 | RS |
| phtalic anhydride | 85-44-9 | RS |
| toluendiisocyanate | 584-84-9 | RS |
| trimellitic anhydride | 552-30-7 | RS |
| 2-aminophenol | 95-55-6 | SS / NRS |
| 2-hydroxtethyl acrylate | 818-61-1 | SS / NRS |
| 2-nitro-1,4-phenylendiamine | 5307-14-2 | SS / NRS |
| formaldehyde | 50-00-0 | SS / NRS |
| geraniol | 106-24-1 | SS / NRS |
| hexylcinnamic aldehyde | 101-86-0 | SS / NRS |
| kathon CG | 96118-96-6 | SS / NRS |
| penicillin G | 61-33-6 | SS / NRS |
| potassium dichromate | 7778-50-9 | SS / NRS |
| 1-butanol | 71-36-3 | NS |
| 4-aminobenzoic acid | 150-13-0 | NS |
| chlorobenzene | 108-90-7 | NS |
| dimethyl formamide | 68-12-2 | NS |
| ethyl vanillin | 121-32-4 | NS |
| isopropanol | 67-63-0 | NS |
| methyl salicylate | 119-36-8 | NS |
| potassium permanganate | 7722-64-7 | NS |
| propylene glycol | 57-55-6 | NS |
| tween 80 | 9005-65-6 | NS |
| zinc sulphate | 7733-02-0 | NS |

RS; Respiratory sensitizer, SS; Skin sensitizer, NRS; Non-respiratory sensitizer, NS; Non-sensitizer.

**Table 2.** Identities of the 28 genes of the GRPS.

| Gene name | Gene Symbol | Entrez ID | Affymetrix ID |
|---|---|---|---|
| *amino-terminal enhancer of split* | AES | 166 | 8032576 |
| *solute carrier family 44 member 2* | SLC44A2 | 57153 | 8025672 |
| *ring finger protein 220* | RNF220 | 55182 | 7900979 |
| *ADAM metallopeptidase domain 8* | ADAM8 | 101 | 7937150 |
| *RAS like proto-oncogene A* | RALA | 5898 | 8132406 |
| *interleukin 7 receptor* | IL7R | 3575 | 8104901 |
| *fascin actin-bundling protein 1* | FSCN1 | 6624 | 8131339 |
| *phosphatidylinositol specific phospholipase C X domain containing 1* | PLCXD1 | 55344 | 8165711 |
| *ZFP36 ring finger protein like 1* | ZFP36L1 | 677 | 7979813 |
| *cytokine receptor-like factor 2* | CRLF2 | 64109 | 8171105 |
| *CCCTC-binding factor* | CTCF | 10664 | 7996593 |
| *family with sequence similarity 30 member A* | FAM30A | 9834 | 7977440 |
| *G protein subunit alpha 15* | GNA15 | 2769 | 8024572 |
| *calcium voltage-gated channel auxiliary subunit alpha2delta 2* | CACNA2D2 | 9254 | 8087691 |
| *MYCN proto-oncogene, bHLH transcription factor* | MYCN | 4613 | 8040419 |
| *arachidonate 5-lipoxygenase activating protein* | ALOX5AP | 241 | 7968344 |
| *versican* | VCAN | 1462 | 8106743 |
| *cathepsin H* | CTSH | 1512 | 7990757 |
| *RAB27B, member RAS oncogene family* | RAB27B | 5874 | 8021301 |

(continued)

| Gene name | Gene Symbol | Entrez ID | Affymetrix ID |
|---|---|---|---|
| *Ral GTPase activating protein catalytic alpha subunit 2* | RALGAPA2 | 57186 | 8065280 |
| *phosphatidylinositol 3-kinase catalytic subunit type* 3 | PIK3C3 | 5289 | 8021015 |
| *ninjurin 1* | NINJ1 | 4814 | 8162455 |
| *nuclear receptor interacting protein 1* | NRIP1 | 8204 | 8069553 |
| *Heterogeneous Nuclear Ribonucleoprotein C (C1/C2)* | HNRNPC | 3183 | 7893129 |
| *HOP homeobox* | HOPX | 84525 | 8100507 |
| *atlastin GTPase 1* | ATL1 | 51062 | 7974270 |
| *oxysterol binding protein like 3* | OSBPL3 | 26031 | 8138613 |
| *RUN and FYVE domain containing 1* | RUFY1 | 80230 | 8110499 |

**Table 3.** Prediction results of external test data sets using the finalized GARDair prediction model.

| Chemical name | True group | Prediction Test set 1 | Prediction Test set 2 | Included in Training Set |
|---|---|---|---|---|
| 2-Mercaptobenzothiazole | NRS | NRS | - | No |
| 4-Hydroxybenzoic acid | NRS | NRS | - | No |
| Benzaldehyde | NRS | NRS | - | No |
| Octanoic acid | NRS | NRS | - | No |
| Chloramine-T hydrate | RS | RS | RS | No |
| Cinnamyl alcohol | NRS | NRS | - | No |
| Diethyl phthalate | NRS | NRS | - | No |
| DNCB | NRS | NRS | NRS | No |
| Eugenol | NRS | NRS | - | No |
| Glycerol | NRS | NRS | - | No |
| Glyoxal | NRS | RS | - | No |
| Isoeugenol | NRS | NRS | - | No |
| Isophorone diisocyanate | RS | RS | - | No |
| Phenol | NRS | NRS | - | No |
| Piperazine | RS | RS | RS | No |
| PPD | NRS | NRS | NRS | No |
| Reactive orange 16 | RS | RS | RS | No |
| Resorcinol | NRS | NRS | - | No |
| Salicylic acid | NRS | RS | - | No |
| SDS | NRS | NRS | - | No |
| Chlorobenzene | NRS | - | NRS | Yes |
| DMSO | NRS | - | NRS | Yes |
| Maleic anhydride | RS | - | RS | Yes |
| Phenyl isocyanate (MDI) | RS | - | RS | Yes |
| Phthalic anhydride | RS | - | RS | Yes |
| Toluene diisocyanate | RS | - | NRS | Yes |
| Trimelitic anhydride | RS | - | RS | Yes |

RS; Respiratory sensitizer, NRS; Non-respiratory sensitizer. False classifications are highlighted.

**Table 4.**

Listed below are details of the algorithm script, written in R code, used to perform the method:
#This code describes the typical usage of the GRPS in its intended application as constituting
#predictors in a computational prediction model. Dependencies on standard functions are
#stored in GARD_GRPS.R.

(continued)

```
# Required files:
# - GARD_GRPS.R
# - raw affymetrix files of test samples in subdir: raw_affy/
# - Annotation of the new data describing the unstimulated samples raw_affy/annotation.rds
# - Historical data stored in trainingset.rds
# Load required dependencies
source('~/GARD_GRPS.R')
# Load Training Data
train = readRDS('~/trainingset.rds')
# Read new data and annotations
new_data = read_raw_affy('~/raw_affy/*.CEL')
new_data_ref = readRDS('~/raw_affy/annotation.rds')
# Normalize the new data
normalized_data = normalize_train_test(train = train, test = new_data, test_reference = new_data_ref)
# Train model on historical data
model = train_svm(normalized_data)
# Predict New Samples
predictions = predict_test_samples(model = model, data=normalized_data)
```

**Table 5. Weightings**

| Gene name | Gene Symbol | Entrez ID | Affymetrix ID | Weight |
|---|---|---|---|---|
| cytokine receptor-like factor 2 | CRLF2 | 64109 | 8171105 | -1.01835933608703 |
| fascin actin-bundling protein 1 | FSCN1 | 6624 | 8131339 | 1.00203258207129 |
| amino-terminal enhancer of split | AES | 166 | 8032576 | -0.937232228971051 |
| arachidonate 5-lipoxygenase activating protein | ALOX5AP | 241 | 7968344 | 0.859616973865753 |
| RAB27B, member RAS oncogene family | RAB27B | 5874 | 8021301 | 0.782688844360711 |
| MYCN proto-oncogene, bHLH transcription factor | MYCN | 4613 | 8040419 | -0.775008003430203 |
| ZFP36 ring finger protein like 1 | ZFP36L1 | 677 | 7979813 | -0.719233666771149 |
| solute carrier family 44 member 2 | SLC44A2 | 57153 | 8025672 | 0.718226173217911 |
| atlastin GTPase 1 | ATL1 | 51062 | 7974270 | 0.699374841646448 |
| family with sequence similarity 30 member A | FAM30A | 9834 | 7977440 | 0.683461721920966 |
| cathepsin H | CTSH | 1512 | 7990757 | -0.65487992465195 |
| ninjurin 1 | NINJ1 | 4814 | 8162455 | -0.577359642405239 |
| Ral GTPase activating protein catalytic alpha subunit 2 | RALGAPA2 | 57186 | 8065280 | 0.552163931377946 |
| ring finger protein 220 | RNF220 | 55182 | 7900979 | -0.551522449893945 |
| oxysterol binding protein like 3 | OSBPL3 | 26031 | 8138613 | -0.538467358395433 |
| calcium voltage-gated channel auxiliary subunit alpha2delta 2 | CACNA2D2 | 9254 | 8087691 | -0.51849673058401 |
| Heterogeneous Nuclear Ribonucleoprotein C (C1/C2) | HNRNPC | 3183 | 7893129 | 0.299399629874934 |
| phosphatidylinositol 3-kinase catalytic subunit type 3 | PIK3C3 | 5289 | 8021015 | -0.256425970684912 |

(continued)

| Gene name | Gene Symbol | Entrez ID | Affymetrix ID | Weight |
|---|---|---|---|---|
| interleukin 7 receptor | IL7R | 3575 | 8104901 | 0.215964226173642 |
| HOP homeobox | HOPX | 84525 | 8100507 | 0.166534308063369 |
| versican | VCAN | 1462 | 8106743 | -0.147007737618858 |
| RAS like proto-oncogene A | RALA | 5898 | 8132406 | -0.101979863027782 |
| RUN and FYVE domain containing 1 | RUFY1 | 80230 | 8110499 | 0.0996656054685292 |
| G protein subunit alpha 15 | GNA15 | 2769 | 8024572 | 0.0794276641913698 |
| ADAM metallopeptidase domain 8 | ADAM8 | 101 | 7937150 | -0.0746172327492091 |
| nuclear receptor interacting protein 1 | NRIP1 | 8204 | 8069553 | 0.0715765479932369 |
| CCCTC-binding factor | CTCF | 10664 | 7996593 | 0.0477003538478608 |
| phosphatidylinositol specific phospholipase C X domain containing 1 | PLCXD1 | 55344 | 8165711 | 0.0263482446344047 |

**Claims**

1. An *in vitro* method for identifying agents capable of inducing respiratory sensitization in a mammal comprising or consisting of the steps of:

   (a) providing a population of dendritic cells or a population of dendritic-like cells;
   (b) exposing the cells provided in step (a) to a test agent; and
   (c) measuring in the cells of step (b) the expression of all of the biomarkers listed in Table A; and

   wherein the expression of the biomarkers measured in step (c) is indicative of the respiratory sensitizing effect of the test agent of step (b).

2. The method according to claim 1 further comprising:

   d) exposing a separate population of the dendritic cells or dendritic-like cells to one or more negative control agent that is not a respiratory sensitizer in a mammal; and
   e) measuring in the cells of step (d) the expression of the biomarkers measured in step (c)

   wherein the test agent is identified as a respiratory sensitizer in the event that the expression of the biomarkers measured in step (e) differs from the expression of the biomarkers measured in step (c); and/or
   wherein the method further comprises:

   f) exposing a separate population of the dendritic cells or dendritic-like cells to one or more positive control agent that is a respiratory sensitizer in a mammal; and
   g) measuring in the cells of step (f) the expression of the biomarkers measured in step (c)

   wherein the test agent is identified as a respiratory sensitizer in the event that the expression of the biomarkers measured in step (f) corresponds to the expression of the biomarkers measured in step (c).

3. The method according to any one of the preceding claims wherein step (c) comprises measuring the expression of a nucleic acid molecule of one or more of the biomarkers; optionally wherein the nucleic acid molecule is a cDNA molecule or an mRNA molecule; optionally wherein measuring the expression of one or more of the biomarkers in step (c) is performed using a method selected from the group consisting of Southern hybridisation, Northern hybridisation, polymerase chain reaction (PCR), reverse transcriptase PCR (RT-PCR), quantitative real-time PCR (qRT-PCR), nanoarray, microarray, macroarray, autoradiography and *in situ* hybridisation; optionally wherein measuring the expression of one or more of the biomarkers in step (c) is determined using a DNA microarray.

4. The method according to any one of the preceding claims wherein measuring the expression of one or more of the biomarkers in step (c) is performed using one or more binding moieties, each capable of binding selectively to a nucleic acid molecule encoding one of the biomarkers identified in Table A; optionally wherein the one or more binding moieties each comprise or consist of a nucleic acid molecule.

5. The method according to Claim 4 wherein the one or more binding moieties each comprise or consist of DNA, RNA, PNA, LNA, GNA, TNA or PMO optionally wherein the binding moiety comprises a detectable moiety; optionally wherein the detectable moiety is selected from the group consisting of: a fluorescent moiety; a luminescent moiety; a chemiluminescent moiety; a radioactive moiety (for example, a radioactive atom); or an enzymatic moiety.

6. The method according to any one of the preceding claims wherein step (c) is performed using an array; optionally wherein the array is a bead-based array; optionally wherein the array is a surface-based array; and/or wherein the array is selected from the group consisting of: macroarray; microarray; nanoarray.

7. The method according to any one of the preceding claims wherein the population of dendritic cells or population of dendritic-like cells comprises or consists of immortal and/or non-naturally occurring cells; and/or wherein the population of dendritic cells or population of dendritic-like cells is a population of dendritic-like cells; optionally wherein the dendritic-like cells are myeloid dendritic-like cells; optionally wherein the myeloid dendritic-like cells are derived from myeloid dendritic cells; optionally wherein the cells derived from myeloid dendritic cells are myeloid leukaemia-derived cells such as those selected from the group consisting of KG-1, THP-1, U-937, HL-60, Monomac-6, AML-193, MUTZ-3, and SenzaCell.

8. The method according to any one of the preceding claims for identifying agents capable of inducing a respiratory hypersensitivity response; and/or wherein the hypersensitivity response is a humoral hypersensitivity response; and/or for identifying agents capable of inducing a type I hypersensitivity response in a mammal; and/or for identifying agents capable of inducing respiratory allergy; and/or wherein the method is indicative of the relative sensitizing potency of the sample to be tested.

9. The method according to any one of the preceding claims wherein the method comprises one or more of the following steps:

(i) cultivating dendritic or dendritic-like cells;
(ii) seeding cells of (i) in one or more well(s), e.g. wells of one or more multi-well assay plates;
(iii) adding to a one or more well(s) of (ii) the agent(s) to be tested;
(iv) adding to one or more separate well(s) of (ii) one or more positive control(s);
(v) adding to one or more separate well(s) of (ii) one or more negative control(s);
(vi) incubating cells in wells of (iii)-(v), preferably for about 24 hours;
(vii) isolating purified total RNA from cells of (vi) and, optionally, convert mRNA into cDNA;
(viii) quantifying expression levels of individual mRNA transcripts from (vii), e.g. using an array, such as an Affymetrix Human Gene 1.0 ST array, and/or a Nanostring code set;
(ix) exporting and normalizing expression data from (viii);
(x) isolating data from (ix) originating from biomarkers of the GARD Prediction Signature (i.e. the biomarkers of Table A);
(xi) applying a prediction model to data from (x), e.g. a frozen SVM model previously established and trained on historical data, e.g. data obtained in Example 1, to predict the respiratory sensitization effect of tested agents(s) and negative/positive control(s).

10. An array for use in the method according to any one of Claims 1-9, the array consisting of binding moieties for all of the biomarkers listed in Table A, wherein the binding moieties of the array comprise one or more binding moieties as defined in any one of Claims 4-5; optionally wherein the array comprises one or more binding moiety for each of the biomarkers defined in any one of the preceding claims.

11. Use of all of the biomarkers listed in Table A for identifying respiratory sensitizing agents *in* vitro according to the method of claim 1.

12. Use of binding moieties with specificity for each biomarker listed in Table A for identifying respiratory sensitizing agents *in* vitro according to the method of claim 1.

13. An analytical kit for use in a method according any one of Claims 1-9 comprising:

(a) an array according to Claim 10; and
(b) (optionally) one or more control agent;
(c) (optionally) instructions for performing the method as defined in any one of Claims 1-9.

**Patentansprüche**

1. Verfahren *in vitro* zur Identifizierung von Mitteln, die in der Lage sind, eine Atemwegssensibilisierung bei einem Säugetier hervorzurufen, umfassend oder bestehend aus den folgenden Schritten:

(a) Bereitstellen einer Population dendritischer Zellen oder einer Population dendritenähnlicher Zellen;
(b) Aussetzen der in Schritt (a) bereitgestellten Zellen gegenüber einem Testmittel; und
(c) Messen der Expression aller in Tabelle A aufgeführten Biomarker in den Zellen von Schritt (b); und wobei die in Schritt (c) gemessene Expression der Biomarker die Atemwegssensibilisierung durch das Testmittel aus Schritt (b) angibt.

2. Verfahren nach Anspruch 1, ferner umfassend:

d) Aussetzen einer separaten Population der dendritischen Zellen oder dendritenähnlichen Zellen gegenüber einem oder mehreren negativen Kontrollmitteln, die keine Atemwegssensibilisatoren in einem Säugetier sind; und
e) Messen der Expression der in Schritt (c) gemessenen Biomarker in den Zellen von Schritt (d), wobei das Testmittel als Atemwegssensibilisierer identifiziert wird, falls die Expression der in Schritt (e) gemessenen Biomarker von der Expression der in Schritt (c) gemessenen Biomarker abweicht; und/oder wobei das Verfahren ferner Folgendes umfasst:

f) Aussetzen einer separaten Population der dendritischen Zellen oder dendritenähnlichen Zellen gegenüber einem oder mehreren positiven Kontrollmitteln, die Atemwegssensibilisatoren in einem Säugetier sind; und
g) Messen der Expression der in Schritt (c) gemessenen Biomarker in den Zellen von Schritt (f), wobei das Testmittel als Atemwegssensibilisierer identifiziert wird, wenn die in Schritt (f) gemessene Expression der Biomarker mit der in Schritt (c) gemessenen Expression der Biomarker übereinstimmt.

3. Verfahren nach einem der vorstehenden Ansprüche, wobei Schritt (c) das Messen der Expression eines Nukleinsäuremoleküls eines oder mehrerer der Biomarker umfasst; optional wobei das Nukleinsäuremolekül ein cDNA-Molekül oder ein mRNA-Molekül ist; optional wobei das Messen der Expression eines oder mehrerer der Biomarker in Schritt (c) unter Verwendung eines Verfahrens durchgeführt wird, das aus der Gruppe ausgewählt ist, bestehend aus Südhybridisierung, Nordhybridisierung, Polymerasekettenreaktion (PCR), revertierter Transkriptase-PCR (RT-PCR), quantitativer Echtzeit-PCR (qRT-PCR), Nanoarray, Microarray, Makroarray, Autoradiographie und Hybridisierung *in situ;* optional wobei das Messen der Expression eines oder mehrerer der Biomarker in Schritt (c) unter Verwendung eines DNA-Mikroarrays bestimmt wird.

4. Verfahren nach einem der vorstehenden Ansprüche, wobei das Messen der Expression eines oder mehrerer Biomarker in Schritt (c) unter Verwendung einer oder mehrerer bindender Einheiten durchgeführt wird, die jeweils in der Lage sind, selektiv an ein Nukleinsäuremolekül zu binden, das einen der in Tabelle A identifizierten Biomarker codiert; wobei optional die eine oder die mehreren bindenden Einheiten jeweils ein Nukleinsäuremolekül umfassen oder aus einem solchen bestehen.

5. Verfahren nach Anspruch 4, wobei die eine oder mehreren bindenden Einheiten jeweils DNA, RNA, PNA, LNA, GNA, TNA oder PMO umfassen oder daraus bestehen, wobei die bindende Einheit optional eine nachweisbare Einheit umfasst, wobei die nachweisbare Einheit ausgewählt ist aus der Gruppe, bestehend aus: einer fluoreszierenden Einheit, einer lumineszierenden Einheit, einer chemilumineszierenden Einheit, einer radioaktiven Einheit (z. B. einem radioaktiven Atom) oder einer enzymatischen Einheit.

6. Verfahren nach einem der vorstehenden Ansprüche, wobei Schritt (c) unter Verwendung eines Arrays durchgeführt wird; optional, wobei der Array ein Array auf der Basis von Kügelchen ist; optional, wobei der Array ein Array auf der Basis von Oberflächen ist; und/oder wobei der Array ausgewählt ist aus der Gruppe, bestehend aus: Makroarray;

Mikroarray; Nanoarray.

7. Verfahren nach einem der vorstehenden Ansprüche, wobei die Population dendritischer Zellen oder die Population dendritischähnlicher Zellen unsterbliche und/oder nicht natürlich vorkommende Zellen umfasst oder daraus besteht; und/oder wobei die Population dendritischer Zellen oder die Population dendritischähnlicher Zellen eine Population dendritischähnlicher Zellen ist; wobei es sich bei den dendritischähnlichen Zellen optional um myeloide dendritischähnliche Zellen handelt; wobei die myeloiden dendritischähnlichen Zellen von myeloiden dendritischen Zellen abgeleitet sind; wobei es sich bei den von myeloiden dendritischen Zellen abgeleiteten Zellen optional um von myeloischer Leukämie abgeleitete Zellen handelt, wie solche, die aus der Gruppe ausgewählt sind, bestehend aus KG-1, THP-1, U-937, HL-60, Monomac-6, AML-193, MUTZ-3, und SenzaCell.

8. Verfahren nach einem der vorstehenden Ansprüche zum Identifizieren von Mitteln, die in der Lage sind, eine Atemwegssensibilisierungsreaktion auszulösen; und/oder wobei die Überempfindlichkeitsreaktion eine humorale Überempfindlichkeitsreaktion ist; und/oder zum Identifizieren von Mitteln, die in der Lage sind, eine Überempfindlichkeitsreaktion vom Typ I bei einem Säugetier auszulösen; und/oder zum Identifizieren von Mitteln, die in der Lage sind, eine Atemwegsallergie auszulösen; und/oder wobei das Verfahren die relative Sensibilisierungspotenz der zu testenden Probe angibt.

9. Verfahren nach einem der vorstehenden Ansprüche, wobei das Verfahren einen oder mehrere der folgenden Schritte umfasst:

(i) Kultivieren von dendritischen oder dendritischähnlichen Zellen;
(ii) Aussäen der Zellen von (i) in eine oder mehrere Vertiefung(en), z. B. Vertiefungen einer oder mehrerer Assay-Platten mit mehreren Vertiefungen;
(iii) Zugeben des (der) zu testenden Mittel(s) in eine oder mehrere Vertiefung(en) von (ii);
(iv) Zugeben in eine oder mehrere separate Vertiefung (en) von (ii) einer oder mehreren positiven Kontrolle(n);
(v) Zugeben in eine oder mehrere separate Vertiefung (en) von (ii) einer oder mehreren negativen Kontrolle(n);
(vi) Inkubieren der Zellen in den Vertiefungen von (iii)-(v), vorzugsweise für etwa 24 Stunden;
(vii) Isolieren der gereinigten Gesamt-RNA aus den Zellen von (vi) und gegebenenfalls Umwandeln der mRNA in cDNA;
(viii) Quantifizieren der Expressionsniveaus einzelner mRNA-Transkripte aus (vii), z. B. unter Verwendung eines Arrays, wie beispielsweise eines Affymetrix Human Gene 1.0 ST Arrays, und/oder eines Nanostring-Code-Satzes;
(ix) Exportieren und Normalisieren von Expressionsdaten aus (viii);
(x) Isolieren von Daten aus (ix), die von Biomarkern der GARD-Vorhersagesignatur stammen (d. h. den Biomarkern aus Tabelle A);
(xi) Anwenden eines Vorhersagemodells auf die Daten aus (x), z. B. eines eingefrorenen SVM-Modells, das zuvor erstellt und auf historischen Daten, z. B. den in Beispiel 1 erhaltenen Daten, trainiert wurde, um die Sensibilisierungswirkung der getesteten Mittel und der negativen/positiven Kontrolle(n) auf die Atemwege vorherzusagen.

10. Array zur Verwendung in dem Verfahren nach einem der Ansprüche 1 bis 9, wobei der Array aus bindenden Einheiten für alle in Tabelle A aufgeführten Biomarker besteht, wobei die bindenden Einheiten des Arrays eine oder mehrere bindende Einheiten, wie in einem der Ansprüche 4 bis 5 definiert, umfassen; wobei das Array optional eine oder mehrere bindende Einheiten für jeden der in einem der vorstehenden Ansprüche definierten Biomarker umfasst.

11. Verwendung aller in Tabelle A aufgeführten Biomarker zum Identifizieren von Atemwegssensibilisierungsmitteln in vitro nach dem Verfahren nach Anspruch 1.

12. Verwendung von bindenden Einheiten mit Spezifität für jeden in Tabelle A aufgeführten Biomarker zum Identifizieren von Atemwegssensibilisierungsmitteln *in vitro* nach dem Verfahren nach Anspruch 1.

13. Analytischer Satz zur Verwendung in einem Verfahren nach einem der Ansprüche 1 bis 9, umfassend:

(a) ein Array nach Anspruch 10; und
(b) (optional) ein oder mehrere Kontrollmittel;
(c) (optional) Anweisungen zur Durchführung des Verfahrens nach einem der Ansprüche 1 bis 9.

**Revendications**

1. Procédé *in vitro* d'identification d'agents capables d'induire une sensibilisation des voies respiratoires chez un mammifère, comprenant ou étant constitué des étapes :

   (a) de fourniture d'une population de cellules dendritiques ou d'une population de cellules de type dendritique ;
   (b) d'exposition des cellules fournies à l'étape (a) à un agent de test ; et
   (c) de mesure dans les cellules de l'étape (b) de l'expression de tous les biomarqueurs répertoriés dans le tableau A ; et

   dans lequel l'expression des biomarqueurs mesurés à l'étape (c) est indicative de l'effet de sensibilisation des voies respiratoires de l'agent de test de l'étape (b).

2. Procédé selon la revendication 1, comprenant également :

   d) l'exposition d'une population distincte des cellules dendritiques ou de cellules de type dendritique à un ou plusieurs agents témoins négatifs qui ne sont pas des sensibilisants des voies respiratoires chez un mammifère ; et
   e) la mesure dans les cellules de l'étape (d) de l'expression des biomarqueurs mesurés à l'étape (c)
   dans lequel l'agent de test est identifié comme un sensibilisant des voies respiratoires dans le cas où l'expression des biomarqueurs mesurés à l'étape (e) diffère de l'expression des biomarqueurs mesurés à l'étape (c) ; et/ou
   dans lequel le procédé comprend également :

      f) l'exposition d'une population distincte des cellules dendritiques ou de cellules de type dendritique à un ou plusieurs agents témoins positifs qui sont des sensibilisants des voies respiratoires chez un mammifère ; et
      g) la mesure dans les cellules de l'étape (f) de l'expression des biomarqueurs mesurés à l'étape (c)
      dans lequel l'agent de test est identifié comme un sensibilisant des voies respiratoires dans le cas où l'expression des biomarqueurs mesurés à l'étape (f) correspond à l'expression des biomarqueurs mesurés à l'étape (c).

3. Procédé selon l'une quelconque des revendications précédentes, dans lequel l'étape (c) comprend la mesure de l'expression d'une molécule d'acide nucléique d'un ou de plusieurs des biomarqueurs ; éventuellement dans lequel la molécule d'acide nucléique est une molécule d'ADNc ou une molécule d'ARNm ; éventuellement dans lequel la mesure de l'expression d'un ou de plusieurs des biomarqueurs à l'étape (c) est réalisée à l'aide d'un procédé choisi dans le groupe constitué par l'hybridation de Southern, l'hybridation de Northern, la réaction en chaîne par polymérase (PCR), la PCR par transcriptase inverse (RT-PCR), la PCR quantitative en temps réel (qRT-PCR), la nanopuce, la micropuce, la macropuce, l'autoradiographie et l'hybridation *in situ* ; éventuellement dans lequel la mesure de l'expression d'un ou de plusieurs des biomarqueurs à l'étape (c) est déterminée à l'aide d'une micropuce à ADN.

4. Procédé selon l'une quelconque des revendications précédentes, dans lequel la mesure de l'expression d'un ou de plusieurs des biomarqueurs à l'étape (c) est réalisée à l'aide d'une ou de plusieurs fractions de liaison, chacune capable de se lier sélectivement à une molécule d'acide nucléique codant pour l'un des biomarqueurs identifiés dans le tableau A ; éventuellement dans lequel les une ou plusieurs fractions de liaison comprennent ou sont chacune constituées d'une molécule d'acide nucléique.

5. Procédé selon la revendication 4, dans lequel les une ou plusieurs fractions de liaison comprennent ou sont constituées chacune d'ADN, d'ARN, de PNA, de LNA, de GNA, de TNA ou de PMO, éventuellement dans lequel la fraction de liaison comprend une fraction détectable ; éventuellement dans lequel la fraction détectable est choisie dans le groupe constitué par : une fraction fluorescente ; une fraction luminescente ; une fraction chimioluminescente ; une fraction radioactive (par exemple, un atome radioactif) ; ou une fraction enzymatique.

6. Procédé selon l'une quelconque des revendications précédentes, dans lequel l'étape (c) est réalisée à l'aide d'une puce ; éventuellement dans lequel la puce est une puce à base de billes ; éventuellement dans lequel la puce est une puce à base de surface ; et/ou dans lequel la puce est choisie dans le groupe constitué de : macropuce ; micropuce ; nanopuce.

7. Procédé selon l'une quelconque des revendications précédentes, dans lequel la population de cellules dendritiques ou la population de cellules de type dendritique comprend ou est constituée de cellules immortelles et/ou non naturelles ; et/ou dans lequel la population de cellules dendritiques ou la population de cellules de type dendritique est une population de cellules de type dendritique ; éventuellement dans lequel les cellules de type dendritique sont des cellules de type dendritique myéloïdes ; éventuellement dans lequel les cellules de type dendritique myéloïdes sont dérivées de cellules dendritiques myéloïdes ; éventuellement dans lequel les cellules dérivées de cellules dendritiques myéloïdes sont des cellules dérivées de la leucémie myéloïde telles que celles choisies dans le groupe constitué de KG-1, THP-1, U-937, HL-60, Monomac-6, AML-193, MUTZ-3 et SenzaCell.

**8.** Procédé selon l'une quelconque des revendications précédentes d'identification d'agents capables d'induire une réponse d'hypersensibilité des voies respiratoire ; et/ou dans lequel la réponse d'hypersensibilité est une réponse d'hypersensibilité humorale ; et/ou d'identification d'agents capables d'induire une réponse d'hypersensibilité de type I chez un mammifère ; et/ou d'identification d'agents capables d'induire une allergie des voies respiratoires ; et/ou dans lequel le procédé est indicatif de la puissance sensibilisante relative de l'échantillon à tester.

**9.** Procédé selon l'une quelconque des revendications précédentes, dans lequel le procédé comprend une ou plusieurs des étapes suivantes :

(i) la cultivation de cellules dendritiques ou de type dendritique ;
(ii) l'ensemencement de cellules de (i) dans un ou plusieurs puits, par exemple les puits d'une ou de plusieurs plaques d'essai multipuits ;
(iii) l'ajout à un ou plusieurs puits (ii) d'agent(s) à tester ;
(iv) l'ajout à un ou plusieurs puits séparés (ii) d'un ou de plusieurs témoins positifs ;
(v) l'ajout à un ou plusieurs puits séparés (ii) d'un ou de plusieurs témoins négatifs ;
(vi) l'incubation de cellules dans les puits de (iii) à (v), de préférence pendant environ 24 heures ;
(vii) l'isolation de l'ARN total purifié des cellules de (vi) et, éventuellement, convertir l'ARNm en ADNc ;
(viii) la quantification de niveaux d'expression de transcrits d'ARNm individuels de (vii), par exemple à l'aide d'une puce, telle qu'une puce Affymetrix Human Gene 1.0 ST, et/ou un ensemble de codes Nanostring ;
(ix) l'exportation et la normalisation de données d'expression à partir de (viii) ;
(x) l'isolation de données de (ix) provenant des biomarqueurs de la signature de prédiction GARD (c'est-à-dire les biomarqueurs du tableau A) ;
(xi) l'application d'un modèle de prédiction aux données de (x), par exemple un modèle SVM congelé précédemment établi et entraîné sur des données historiques, par exemple des données obtenues dans l'exemple 1, pour prédire l'effet de sensibilisation des voies respiratoires des agents testés et des témoins négatifs/positifs.

**10.** Puce destinée à être utilisée dans le procédé selon l'une quelconque des revendications 1 à 9, la puce étant constituée de fractions de liaison pour tous les biomarqueurs répertoriés dans le tableau A, dans laquelle les fractions de liaison de la puce comprennent une ou plusieurs fractions de liaison telles que définies selon l'une quelconque des revendications 4 à 5 ; éventuellement dans laquelle la puce comprend une ou plusieurs fractions de liaison pour chacun des biomarqueurs définis selon l'une quelconque des revendications précédentes.

**11.** Utilisation de tous les biomarqueurs répertoriés dans le tableau A pour l'identification d'agents sensibilisants des voies respiratoires *in vitro* selon le procédé selon la revendication 1.

**12.** Utilisation de fractions de liaison avec une spécificité pour chaque biomarqueur répertorié dans le tableau A pour l'identification d'agents sensibilisants des voies respiratoires *in vitro* selon le procédé selon la revendication 1.

**13.** Kit d'analyse destiné à être utilisé dans un procédé selon l'une quelconque des revendications 1 à 9, comprenant :

(a) une puce selon la revendication 10 ; et
(b) (éventuellement) un ou plusieurs agents témoins ;
(c) (éventuellement) des instructions pour la réalisation du procédé tel que défini selon l'une quelconque des revendications 1 à 9.

FIG. 1

FIG. 2

FIG. 3

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- WO 2013160882 A, Forreryd
- WO 2016083604 A
- WO 2018122219 A
- EP 39578 A
- US 5856090 A
- WO 9837186 A
- US 4376110 A, David
- US 4486530 A

## Non-patent literature cited in the description

- **CHARYA et al.** *Toxicology Letters*, 2018, 195
- **ROBINSON et al.** *BMC Bioinformatics*, 2007, vol. 8 (1), 449
- **SCHERVISH, MARK J.** A Review of Multivariate Analysis. *Statistical Science*, November 1987, vol. 2 (4), 396-413
- **BURGES**. *Data Mining and Knowledge Discovery*, 1998, vol. 2, 121-167
- **FRAKER et al.** *Biochem. Biophys. Res. Comm.*, 1978, vol. 80, 49-57
- **J-F CHATAL**. Monoclonal Antibodies in Immunoscintigraphy. CRC Press, 1989
- **WINTER ; MILSTEIN**. *Nature*, 1991, vol. 349, 293-299
- **COLLETT et al.** *Methods*, 2005, vol. 37, 4-15
- **CLACKSON et al.** *Nature*, 1991, vol. 352, 624-628
- **MARKS et al.** *J Mol Biol*, 1991, vol. 222 (3), 581-97
- **SMITH**. *Science*, 1985, vol. 228 (4705), 1315-7
- **SANTI et al.** *J Mol Biol*, 2000, vol. 296 (2), 497-508
- **GUNNERIUSSON et al.** *Appl Environ Microbiol*, 1999, vol. 65 (9), 4134-40
- **KENAN et al.** *Methods Mol Biol*, 1999, vol. 118, 217-31
- **KIEKE et al.** *Proc Natl Acad Sci USA*, 1999, vol. 96 (10), 5651-6
- **HANES ; PLUCKTHUN**. *Proc Natl Acad Sci USA*, 1997, vol. 94 (10), 4937-42
- **HE ; TAUSSIG**. *Nucleic Acids Res*, 1997, vol. 25 (24), 5132-4
- **NEMOTO et al.** *FEBS Lett*, 1997, vol. 414 (2), 405-8
- **DAUGHERTY et al.** *Protein Eng*, 1998, vol. 11 (9), 825-32
- **DAUGHERTY et al.** *Protein Eng*, 1999, vol. 12 (7), 613-21
- **SHUSTA et al.** *J Mol Biol*, 1999, vol. 292 (5), 949-56
- **MORRISON et al.** *Proc. Natl. Acad. Sci. USA*, 1984, vol. 81, 6851-6855
- **BETTER et al.** *Science*, 1988, vol. 240, 1041
- **SKERRA et al.** *Science*, 1988, vol. 240, 1038
- **BIRD et al.** *Science*, 1988, vol. 242, 423
- **HUSTON et al.** *Proc. Natl. Acad. Sci. USA*, 1988, vol. 85, 5879
- **WARD et al.** *Nature*, 1989, vol. 341, 544
- **H ZOLA**. Monoclonal Antibodies: A manual of techniques. CRC Press, 1988
- **J G R HURRELL**. Monoclonal Hybridoma Antibodies: Techniques and applications. CRC Press, 1982
- **JENKINS, R.E. ; PENNINGTON, S.R.** *Proteomics*, 2001, vol. 2, 13-29
- **LAL et al.** *Drug Discov Today*, 2002, vol. 7 (18), S143-9
- **STEINMAN et al.** *Ann. Rev. Immunol.*, 1991, vol. 9, 271
- **CHAN-YEUNG ; MALO**. Aetiological agents in occupational asthma. *European Respiratory Journal*, 1994
- **DEARMAN et al.** Classification of chemical allergens according to cytokine secretion profiles of murine local lymph node cells. *Journal of Applied Toxicology*, 1997
- **DEARMAN et al.** Inter-relationships between different classes of chemical allergens. *Journal of Applied Toxicology*, 2011
- **DEARMAN et al.** Inter-relationships between different classes of chemical allergens. *Journal of Applied Toxicology*, 2012
- **FORRERYD et al.** Prediction of chemical Respiratory sensitizers using GARD, a novel in vitro assay based on a genomic biomarker signature. *PLoS One*, 2015, vol. 10 (3)
- **FORRERYD et al.** From genome-wide arrays to tailor-made biomarker readout - Progress towards routine analysis of skin sensitizing chemicals with GARD. *Toxicology in vitro*, 2016

- **GEISS et al.** Direct multiplexed measurement of gene expression with color-coded probe pairs. *Nature Biotechnology*, 2008
- **ISOLA et al.** Chemical respiratory allergy and occupational asthma: what are the key areas of uncertainty?. *Journal of Applied Toxicology*, 2008
- **JOHANSSON et al.** A genomic biomarker signature can predict skin sensitizers using a cell-based in vitro alternative to animal tests. *BMC Genomics*, 2011
- **KIMBER et al.** Chemical respiratory allergy: role of IgE antibody and relevance of route of exposure. *Toxicology*, 2002
- **KIMBER et al.** Chemical allergy: translating biology into hazard characterization. *Toxicological Sciences*, 2011
- **KIMBER et al.** Chemical respiratory allergy: reverse engineering an adverse outcome pathway. *Toxicology*, 2014
- **LALKO et al.** The direct peptide reactivity assay: selectivity of chemical respiratory allergens. *Toxicological Sciences*, 2012
- **PAUL** ; **ZHU**. How are Th2-type immune responses initiated and amplified. *Nature Reviews Immunology*, 2010
- **SOUMELIS et al.** Human epithelial cells trigger dendritic cell-mediated allergic inflammation by producing TSLP. *Nat Immunol.*, 2002
- **SULLIVAN et al.** An Adverse Outcome Pathway for Sensitization of the Respiratory Tract by Low-Molecular-Weight Chemicals: Building Evidence to Support the Utility of In Vitro and In Silico Methods in a Regulatory Context. *Applied in vitro Toxicology*, 2017